# EUROPÄISCHE PATENTSCHRIFT

(11) **EP 4 119 081 B1**
(45) Veröffentlichungstag und Bekanntmachung des Hinweises auf die Patenterteilung: **23.09.2026**
(21) Anmeldenummer: 22189291.2
(22) Anmeldetag: 15.02.2017
(51) Int. Cl.: A61B 18/12, A61B 18/00, H01R 24/40, H01R 24/76

(54) **CHIRURGIEGERÄTE-MEHRFACHBUCHSE, ELEKTROCHIRURGIE-HOCHFREQUENZGENERATOR, ELEKTROCHIRURGIEGERÄTESTECKER UND ELEKTROCHIRURGISCHES SYSTEM**
SURGICAL DEVICE MULTIPLE SOCKET ASSEMBLY, ELECTROSURGICAL HIGH FREQUENCY GENERATOR, ELECTROSURGICAL INSTRUMENT PLUG, AND ELECTROSURGICAL SYSTEM
PRISE MULTIPLE POUR APPAREILS CHIRURGICAUX, GÉNÉRATEUR HAUTE FRÉQUENCE D'ÉLECTROCHIRURGIE, CONNECTEUR D'APPAREIL D'ÉLECTROCHIRURGIE ET SYSTÈME D'ÉLECTROCHIRURGIE

(30) Priorität: 15.02.2016 DE 102016102640
(43) Veröffentlichungstag der Anmeldung: 18.01.2023
(62) Dokumentnummer(n) der früheren Anmeldung(en) nach Art. 76 EPÜ: 17708444.9 / 3 416 577
(73) Patentinhaber: Olympus Winter & Ibe GmbH, 22045 Hamburg (DE)
(72) Erfinder: Holub, Benjamin, 12107 Berlin (DE); Arnold, Mario, 12157 Berlin (DE)
(74) Vertreter: Eisenführ Speiser

(56) Entgegenhaltungen:
- EP-A1- 2 537 480
- WO-A1-2009/071256
- WO-A2-02/085230
- AU-A1- 2005 200 527
- DE-A1- 102007 061 483
- US-A- 5 817 092
- US-A1- 2004 097 117
- US-A1- 2004 097 912
- US-A1- 2012 095 453

## Beschreibung

Die Erfindung betrifft eine Chirurgiegeräte-Mehrfachbuchse für einen Hochfrequenzgenerator zum Anschluss eines elektrochirurgischen Instruments, einen Elektrochirurgie-Hochfrequenzgenerator, einen Elektrochirurgiegerätestecker sowie ein elektrochirurgisches System. Darüber hinaus betrifft die Erfindung ein Kabel für ein elektrochirurgisches Instrument und ein Elektrochirurgisches Instrument.

Elektrochirurgische Instrumente werden in der Chirurgie insbesondere zum monopolaren oder bipolaren Schneiden und/oder Koagulieren biologischen Gewebes eingesetzt. Die Energie für das elektrochirurgische Instrument wird von einem Hochfrequenz(HF)-Generator bereitgestellt. An einem derartigen Hochfrequenzgenerator können unterschiedliche elektrochirurgische Instrumente angeschlossen werden, die jeweils die gleichen oder unterschiedliche Funktionalitäten für den Anwender bereitstellen. Die angeschlossenen elektrochirurgischen Instrumente werden von dem Hochfrequenzgenerator Hochfrequenzenergie versorgt.

Damit die elektrochirurgischen Instrumente an den Hochfrequenzgenerator angeschlossen werden können, bieten derzeit eingesetzte Hochfrequenzgeneratoren im Allgemeinen unterschiedliche Buchsen für die verschiedenartigen Stecker der elektrochirurgischen Instrumente. Daher ist es in der Praxis üblich, dass die Hochfrequenzgeneratoren unterschiedliche Buchsen aufweisen und somit keine Adapter für die einzelnen elektrochirurgischen Instrumente vorhanden sein müssen. Chirurgiegerätesteckersysteme sind beispielsweise aus der DE 10 2007 061 483 A1 bekannt. In den bekannten Gerätesteckersystemen werden an der Buchse Öffnungen, insbesondere für Kontaktstifte, bereitgestellt, wobei die Funktionalität der Buchse, beispielsweise durch Codieröffnungen, erweitert wird. Dadurch können neben der Stromübertragung beispielsweise auch Daten übertragen werden.

US-A-5 817 092 offenbart eine Chirurgiegeräte-Mehrfachbuchse nach dem Oberbegriff des Anspruchs 1.

Die existierenden Buchsen, Vorrichtungen sowie Verfahren zum Anschließen von elektrochirurgischen Instrumenten an Hochfrequenzgeneratoren bieten verschiedene Vorteile, jedoch sind weitere Verbesserungen wünschenswert.

Es ist daher eine Aufgabe der vorliegenden Erfindung, eine Chirurgiegeräte-Mehrfachbuchse für einen Hochfrequenzgenerator zum Anschluss eines elektrochirurgischen Instruments, einen Elektrochirurgie-Hochfrequenzgenerator, einen Elektrochirurgiegerätestecker und ein elektrochirurgisches System anzugeben, welche gegenüber existierenden Buchsen, Vorrichtungen und Verfahren verbessert sind. Es ist insbesondere eine Aufgabe der vorliegenden Erfindung, eine Chirurgiegeräte-Mehrfachbuchse für einen Hochfrequenzgenerator zum Anschluss eines elektrochirurgischen Instruments, einen Elektrochirurgie-Hochfrequenzgenerator, einen Elektrochirurgiegerätestecker und ein elektrochirurgisches System bereitzustellen, welche die Kompatibilität der verwendeten Vorrichtungen erhöhen und/oder eine verbesserte Sicherheitssituation vor einem, während eines und nach einem Chirurgieprozess gewährleisten.

Diese Aufgabe wird gemäß einem ersten Aspekt der Erfindung gelöst durch eine Chirurgiegeräte-Mehrfachbuchse gemäß Anspruch 1.

Die erfindungsgemäße Chirurgiegeräte-Mehrfachbuchse beruht unter anderem auf der Erkenntnis, dass eine Vielzahl unterschiedlicher elektrochirurgischer Instrumente und zudem unterschiedlicher Stecker für diese elektrochirurgischen Instrumente in der Praxis vorhanden ist. Dies resultiert darin, dass die Hochfrequenzgeneratoren mit einer Vielzahl unterschiedlicher Buchsen bereitgestellt werden, so dass die elektrochirurgischen Instrumente, aufweisend unterschiedliche Stecker, an den Hochfrequenzgenerator angeschlossen werden können. In Folge dessen ist eine Verringerung der Baugröße der Hochfrequenzgeneratoren lediglich eingeschränkt möglich, da eine nicht unwesentliche Fläche des Hochfrequenzgenerators für die Zurverfügungstellung der Buchsen vorgehalten werden muss. Insbesondere die für den Benutzer zugänglichen Front- und Seitenflächen des Hochfrequenzgenerators können nur eingeschränkt verkleinert oder für andere Zwecke, wie beispielsweise Displays oder ähnliches verwendet werden, da die Zurverfügungstellung der unterschiedlichen Buchsen viel Raum einnimmt.

Eine Verringerung dieser Fläche ist lediglich mit einer Einschränkung der Kompatibilität des Hochfrequenzgenerators möglich, nämlich indem die Anzahl der zur Verfügung stehenden Buchsen reduziert wird. Dies hat jedoch zur Folge, dass eine geringere Anzahl an elektrochirurgischen Instrumenten an den Hochfrequenzgenerator angeschlossen werden kann. Insbesondere wird der Anwender in seiner Freiheit zur Auswahl des Instrumentenherstellers eingeschränkt, da sich die Hersteller in der Regel auf einen bestimmten Stecker für ihre elektrochirurgischen Instrumente fokussieren. Eine weitere Möglichkeit zur Erhöhung der Kompatibilität ist die Bereitstellung von Adaptern, wobei deren Anwendung im Chirurgiebetrieb mit einer Vielzahl an Nachteilen verbunden ist, wie beispielsweise dem Verlorengehen und der Nutzung eines nicht-geeigneten Adapters für ein spezifisches elektrochirurgisches Instrument oder die Einschränkung der Datenübertragung durch den Adapter.

Durch die Kombination eines Koaxialbuchsenelements mit mindestens einer Kontaktöffnung ermöglicht die Chirurgiegeräte-Mehrfachbuchse den Anschluss sowohl von Koaxialsteckern als auch von Steckern mit zwei Kontaktstiften, deren Abstand dem Abstand der inneren Öffnung des Koaxialbuchsenelements von der Kontaktöffnung entspricht. Damit ermöglicht die Chirurgiegeräte-Mehrfachbuchse bereits die Kombination von zwei bislang getrennten Buchsen und damit die Aufnahme zweiter unterschiedlicher Stecker in ein und derselben Buchse. Die Aufnahme unterschiedlicher Stecker in der Chirurgiegeräte-Mehrfachbuchse erfolgt dabei vorzugsweise nicht gleichzeitig, sondern jeweils dann, wenn ein der jeweilig Stecker benötigt wird, wobei der nicht benötigte Stecker vorzugsweise zuvor aus der Chirurgiegeräte-Mehrfachbuchse entfernt wird.

Der jeweilige in die Chirurgiegeräte-Mehrfachbuchse einzuführende Stecker ist im vorliegenden Anwendungsfall vorzugsweise ein Bestandteil eines elektrochirurgischen Instruments, um letztgenanntes an einen Hochfrequenzgenerator anzuschließen. Die Chirurgiegeräte-Mehrfachbuchse weist unter anderem die Koaxialbuchse auf, die insbesondere ausgebildet ist, ein Koaxialsteckerelement aufzunehmen. Eine Koaxialbuchse dient insbesondere der Aufnahme eines Koaxialkabels sowie zur Bildung einer lösbaren Verbindung zwischen einem Ende des Kabels, an dem ein Koaxialsteckerelement angeordnet ist, und der Koaxialbuchse.

Die Koaxialbuchse umfasst eine innere und eine äußere Hülse, die koaxial und vorzugsweise ineinander geschoben angeordnet sind. Der Außendurchmesser der inneren Hülse weist vorzugsweise eine geringere Abmessung auf als der Innendurchmesser der äußeren Hülse. Der Innendurchmesser der äußeren Hülse ist vorzugsweise größer als der Außendurchmesser der inneren Hülse. Der Außendurchmesser der äußeren Hülse weist vorzugsweise die doppelte Größe auf als der Außendurchmesser der inneren Hülse. Ferner liegen die Stirnseiten der zwei Hülsen an dem proximalen Ende der Koaxialbuchse vorzugsweise im Wesentlichen in einer gemeinsamen Ebene.

Zwischen der inneren und der äußeren Hülse ist eine äußere, ringförmige Koaxialbuchsenöffnung gebildet. Im inneren der inneren Hülse ist eine innere Koaxialbuchsenöffnung gebildet.

Die Koaxialbuchse ist ausgebildet, ein Koaxialsteckerelement aufzunehmen wobei das Koaxialsteckerelement einen inneren Kontaktstift und einen äußeren Kontaktring aufweist. Der äußere Kontaktring weist eine innere Aussparung auf, in der der innere Kontaktstift angeordnet ist. Der äußere Kontaktring hat demnach vollständig oder abschnittsweise entlang seiner axialen Erstreckung die Geometrie einer Hülse. Somit ist bei der richtigen Wahl der einzelnen Abmessungen eine koaxiale Steckverbindung zwischen der Koaxialbuchse und dem Koaxialsteckerelement möglich, indem der innere Kontaktstift in die innere Koaxialbuchsenöffnung und der äußere Kontaktring in die äußere, ringförmige Koaxialbuchsenöffnung eingeführt werden. Die so erhaltene koaxiale Steckverbindung hat die Vorteile einer sehr geringen elektromagnetischen Beeinflussung und Abstrahlung sowie einer guten elektrischen Abschirmung. Ferner besteht die Möglichkeit zur Nutzung der Koaxialbuchse als Kontaktöffnung, indem ein Kontaktstift in die innere Koaxialbuchsenöffnung der Koaxialbuchse geführt wird.

Die Chirurgiegeräte-Mehrfachbuchse weist ferner die erste Kontaktöffnung und/oder die zweite Kontaktöffnung auf, die jeweils ausgebildet sind, einen Kontaktstift aufzunehmen. Die erste und/oder zweite Kontaktöffnung weist dabei einen vorzugsweisen kreisrunden Querschnitt auf. Alternativ vorzugsweise weist die erste und/oder zweite Kontaktöffnung einen ovalen oder eckigen Querschnitt auf, wobei die korrespondierenden Kontaktstifte dann vorzugsweise ebenfalls eine derartige Geometrie aufweisen.

Die erste und/oder zweite Kontaktöffnung weist ein Anschlussende auf, an dem ein Kontaktstift eingeführt werden kann. An diesem Anschlussende weist die erste und/oder zweite Kontaktöffnung einen definierten Durchmesser auf.

Die Koaxialbuchse sowie die erste und/oder zweite Kontaktöffnung sind erfindungsgemäß an einer einzelnen Chirurgiegeräte-Mehrfachbuchse angeordnet. Diese Anordnung ermöglicht den Anschluss von unterschiedlichen Steckern an die Mehrfachbuchse. Die Stecker können sich beispielsweise in der Anzahl und/oder Art und/oder der Beabstandung der Kontaktelemente, wie beispielsweise Kontaktstifte und/oder Kontaktringe, unterscheiden. Beispielsweise können sich die Stecker hinsichtlich der Kontaktelemente für das Koaxialsteckerelement unterscheiden. Weiterhin besteht die Möglichkeit, dass sich die Stecker hinsichtlich der Kontaktstifte, die ausgebildet sind, um in die erste und/oder zweite Kontaktöffnung eingeführt zu werden, unterscheiden, beispielsweise hinsichtlich des Durchmessers und/oder hinsichtlich der Geometrie.

Die Koaxialbuchse hat bei der erfindungsgemäßen Chirurgiegeräte-Mehrfachbuchse zweierlei Funktionen. Einerseits ist es die Funktion der Koaxialbuchse, ein Koaxialsteckerelement aufzunehmen. Andererseits ist es Aufgabe der Koaxialbuchse, einen üblichen Kontaktstift in der inneren Koaxialbuchsenöffnung, die durch die innere Hülse ausgebildet wird, aufzunehmen. Somit besteht die Möglichkeit, dass ein Stecker mit zwei Kontaktstiften derart in die Chirurgiegeräte-Mehrfachbuchse eingeführt werden kann, dass ein Kontaktstift in die innere Koaxialbuchsenöffnung und der andere Kontaktstift in einer der Kontaktöffnungen eingreift. Durch diese doppelte Nutzung der Koaxialbuchse kann die Gesamtanzahl der notwendigen Kontaktöffnungen an der Chirurgiegeräte-Mehrfachbuchse reduziert und die Flächenausnutzung und Kompatibilität optimiert werden.

Es hat sich ferner vorteilhaft herausgestellt, dass bei der erfindungsgemäßen Anordnung der Kontaktöffnung(en) sowie der Koaxialbuchse das Risiko durch nicht sachgemäß angeschlossene elektrochirurgische Instrumente reduziert werden kann. Dies ist insbesondere dadurch zu begründen, dass an der erfindungsgemäßen Chirurgiegeräte-Mehrfachbuchse lediglich ein einzelnes elektrochirurgisches Instrument angeschlossen werden kann. Ein ungewollter gleichzeitiger Anschluss von möglicherweise nicht kompatiblen Geräten, was zu einer Gefährdung des Personals und/oder des Patienten führen könnte, kann somit ausgeschlossen werden. Auch bei einem Wechsel von Instrumenten wird somit sichergestellt, dass ein neues Instrument erst in Betrieb genommen werden kann, wenn das andere Instrument sicher vom Hochfrequenzgenerator getrennt wurde, was ebenfalls die Sicherheit für Personal und Patienten erhöht. Zusätzliche Überprüfungsschritte, ob keine anderen, möglicherweise inkompatiblen Geräte angeschlossen sind, können bei Hochfrequenzgeneratoren mit der Chirurgiegeräte-Mehrfachbuchse entfallen, so dass die Behandlungszeiten verkürzt werden können.

Als ein weiterer Vorteil hat sich ferner herausgestellt, dass durch die erfindungsgemäße Anordnung der Koaxialbuchse sowie der ersten und/oder zweiten Kontaktöffnung in bzw. an der Chirurgiegeräte-Mehrfachbuchse die Möglichkeit ergeben hat, eine Vielzahl unterschiedlicher elektrochirurgischer Instrumente anzuschließen. Der Anwender wird dadurch in die Lage versetzt, zwischen einer Vielzahl am Markt befindlicher elektrochirurgischer Instrumente auszuwählen. In einer bevorzugten Ausführungsform der Chirurgiegeräte-Mehrfachbuchse umfasst diese eine Positionierungsöffnung, die ausgebildet ist, einen Positionierungsstift aufzunehmen. Vorzugsweise bietet die Positionierungsöffnung in der Regel keine elektrische Funktion bzw. keine primäre elektrische Funktion, sondern dient vor allem der sicheren und richtigen Positionierung des Steckers in der gewünschten Position in der Chirurgiegeräte-Mehrfachbuchse. Insbesondere bei Steckern, die ohne einen Positionierungsstift mehr als eine Möglichkeit der Positionierung in der Buchse zulassen würden, sind ein Positionierungsstift und eine entsprechende Positionierungsöffnung von Vorteil, um eine ein-eindeutige Positionierung des Steckers in der Chirurgiegeräte-Mehrfachbuchse zu erreichen.

Entlang einer axialen Erstreckung ist der Querschnitt der Positionierungsöffnung vorzugsweise im Wesentlichen kreisrund. Es besteht ferner die Möglichkeit, dass die Positionierungsöffnung einen ovalen oder eckigen Querschnitt aufweist, wobei insbesondere ein rechteckiger und/oder viereckiger Querschnitt bevorzugt ist.

Die Abmessungen dieses Querschnitts, orthogonal zu einer longitudinalen Mittelachse, beispielsweise der Durchmesser bei einer kreisrunden Öffnung oder die Kantenabmessungen bei einer eckigen Geometrie, sind vorzugsweise konstant entlang der axialen Erstreckung der Positionierungsöffnung. Diese Abmessungen können jedoch auch entlang der axialen Erstreckung der Positionierungsöffnung variieren, um sich beispielsweise zum Inneren der Chirurgiegeräte-Mehrfachbuchse hin zu reduzieren. Durch eine derartige Gestaltung besteht die Möglichkeit, dass die Positionierungsfunktion der Positionierungsöffnung weiter verbessert wird.

Das Vorsehen einer Positionierungsöffnung sowie eines korrespondierenden Positionierungsstifts an einem Stecker kann den Verschleiß an den elektrischen Kontakten, in diesem Fall der Koaxialbuchse sowie der ersten und/oder zweiten Kontaktöffnung, reduzieren.

Die Erfindung sieht vor, dass die Koaxialbuchse, die erste Kontaktöffnung und die zweite Kontaktöffnung derart angeordnet sind, dass eine gerade Linie Mittelpunkte der Koaxialbuchse, der ersten Kontaktöffnung und der zweiten Kontaktöffnung verbindet. Diese Ausführungsform sieht demnach vor, dass die Koaxialbuchse und die Kontaktöffnung oder die Kontaktöffnungen in einer Flucht angeordnet sind. Dadurch, dass die Linie die Mittelpunkte der Koaxialbuchse sowie der ersten und zweiten Kontaktöffnung verbindet, wird deutlich, dass diese Öffnungen in einer Flucht angeordnet sind. Eine derartige Anordnung ermöglicht insbesondere eine kompaktere Bauweise der Chirurgiegeräte-Mehrfachbuchse und damit wiederum auch eine kompaktere Bauweise des gesamten Hochfrequenzgenerators.

In der Erfindung ist vorgesehen, dass ein Mittelpunkt der Positionierungsöffnung beabstandet zu einer geraden Linie verbindend die Mittelpunkte der Koaxialbuchse, der ersten Kontaktöffnung und der zweiten Kontaktöffnung angeordnet ist. Vorzugsweise verläuft die gerade Linie verbindend die Mittelpunkte der Koaxialbuchse, der ersten Kontaktöffnung und/oder der zweiten Kontaktöffnung nicht durch den Mittelpunkt der Positionierungsöffnung. Die Positionierungsöffnung ist daher vorzugsweise nicht in einer Flucht mit den elektrisch wirkenden Anschlüssen der Chirurgiegeräte-Mehrfachbuchse. Diese nicht in der Flucht angeordnete Positionierungsöffnung hat den besonderen Vorteil, dass dadurch eine sichere und eindeutige Positionierung eines Steckers verbessert wird.

Die Erfindung sieht vor, dass die Positionierungsöffnung zwischen der ersten oder zweiten Kontaktöffnung und der Koaxialbuchse außermittig angeordnet ist. Diese Ausführungsform sieht demnach vor, dass die Positionierungsöffnung nicht auf der geraden Linie, die die Mittelpunkte der Kontaktöffnung oder der Kontaktöffnungen sowie der Koaxialbuchse verbindet, liegt. Ferner sieht diese Ausführungsform vor, dass die Positionierungsöffnung nicht in der Mitte zwischen der Koaxialbuchse und der dazu benachbarten Kontaktöffnung liegt, so dass die Beabstandung zur Koaxialbuchse eine größere oder geringere Abmessung aufweist als die Beabstandung zu der nächstliegenden Kontaktöffnung. Orthogonal zu der geraden Linie, verbindend die Mittelpunkte der Koaxialbuchse, der ersten Kontaktöffnung und/oder der zweiten Kontaktöffnung, besteht damit die Möglichkeit einer sehr geringen Abmessung der Chirurgiegeräte-Mehrfachbuchse, wodurch der Gesamtflächenbedarf reduziert ist und die Bauform des Hochfrequenzgenerators positiv beeinflusst werden kann. Darüber hinaus bietet diese Ausführungsform den Vorteil eines kompakten Steckers.

Gemäß einer weiteren bevorzugten Ausführungsform der Chirurgiegeräte-Mehrfachbuchse ist vorgesehen, dass die erste und die zweite Kontaktöffnung benachbart zueinander angeordnet sind. Die erste und die zweite Kontaktöffnung sind demnach derart angeordnet, dass keine weiteren Öffnungen oder anderen relevanten Bestandteile der Chirurgiegeräte-Mehrfachbuchse zwischen der ersten und der zweiten Kontaktöffnung angeordnet sind. Insbesondere beinhaltet diese Ausführungsform, dass auf einer Verbindungslinie, die die Mittelachsen der ersten und zweiten Kontaktöffnung verbinden, weder die Koaxialbuchse noch gegebenenfalls die Positionierungsöffnung angeordnet ist. Nichtsdestotrotz resultiert die benachbarte Anordnung der Kontaktöffnung vorzugsweise darin, dass beide Öffnungen weiterhin integral bestehen bleiben, so dass die Umwandungen der Kontaktöffnungen keine Überlappung bzw. Überschneidungen aufweisen.

Gemäß einer weiteren besonders bevorzugten Ausführungsform der Chirurgiegeräte-Mehrfachbuchse, ist vorgesehen, dass ein Abstand zwischen einer Mittelachse der ersten Kontaktöffnung und einer Mittelachse der zweiten Kontaktöffnung um ein Vielfaches kleiner ist als ein Abstand zwischen der Mittelachse der ersten Kontaktöffnung und einer Mittelachse der Koaxialbuchse, und/oder ein Abstand zwischen der Mittelachse der zweiten Kontaktöffnung und der Mittelachse der Koaxialbuchse, und/oder ein Abstand zwischen einer Mittelachse der Positionierungsöffnung und der Mittelachse der Koaxialbuchse, wobei vorzugsweise der Abstand zwischen der Mittelachse der ersten Kontaktöffnung und der Mittelachse der Koaxialbuchse größer ist als der Abstand zwischen der Mittelachse der zweiten Kontaktöffnung und der Mittelachse der Koaxialbuchse und ferner vorzugsweise der Abstand zwischen der Mittelachse der zweiten Kontaktöffnung und der Mittelachse der Koaxialbuchse größer ist als der Abstand zwischen der Mittelachse der Positionierungsöffnung und der Mittelachse der Koaxialbuchse.

Diese Anordnung resultiert darin, dass es einen Bereich gibt, in dem die Koaxialbuchse angeordnet ist, und einen zweiten Bereich, in dem die Kontaktöffnungen angeordnet sind. Während beide Bereiche eine vergleichsweise kleine Erstreckung aufweisen, ist eine im Vergleich zur Erstreckung dieser Bereiche große Beabstandung dieser beiden Bereiche voneinander vorhanden. Darüber hinaus resultiert diese Anordnung vorzugsweise darin, dass die Positionierungsöffnung im Bereich oder in der Nähe des Bereichs der ersten und/oder zweiten Kontaktöffnung angeordnet ist und eher weiter beabstandet von der Koaxialbuchse ist. Bevorzugt ist, dass der Abstand der ersten Kontaktöffnung zur Koaxialbuchse die größte Beabstandung zwischen zwei Öffnungen an der Chirurgiegeräte-Mehrfachbuchse aufweist. Darüber hinaus ist es in dieser Ausführungsform bevorzugt, dass die Positionierungsöffnung zwischen einer der Kontaktöffnungen und der Koaxialbuchse angeordnet ist. Ferner kann es bevorzugt sein, das die Positionierungsöffnung zwischen dem Bereich, in dem Koaxialbuchse angeordnet ist, und dem Bereich, in dem die Kontaktöffnungen angeordnet sind, angeordnet ist.

Eine derartige Anordnung der Koaxialbuchse sowie der Kontaktöffnungen ist insbesondere dahingehend vorteilhaft, dass hierdurch die Möglichkeit eröffnet, wird unterschiedliche am Markt verfügbare Stecker mit der Chirurgiegeräte-Mehrfachbuchse zu verbinden. Insbesondere ist es möglich, einen Stecker mit zwei Kontaktstiften mit einer ersten Beabstandung zu verwenden und ferner einen weiteren Stecker mit zwei Kontaktstiften zu verwenden, die einen zweiten Abstand voneinander aufweisen, wobei der zweite Abstand beispielsweise eine geringere Abmessung aufweist als der erste Abstand. Zudem besteht die Möglichkeit, Stecker der zuvor genannten Bauweisen zu verwenden, der zusätzlich einen Positionierungsstift aufweist, der in die Positionierungsöffnung der Chirurgiegeräte-Mehrfachbuchse eingeführt werden kann und somit die richtige Positionierung des Steckers in der Chirurgiegeräte-Mehrfachbuchse sicherstellt.

In einer weiteren Ausführungsform der Chirurgiegeräte-Mehrfachbuchse ist vorgesehen, dass der Abstand zwischen der Mittelachse der ersten Kontaktöffnung und der Mittelachse der zweiten Kontaktöffnung maximal das 10-fache des Durchmessers der ersten und/oder zweiten Kontaktöffnung beträgt. Besonders bevorzugt ist ein Abstand von maximal des 7,5-fachen des Durchmessers, darüber hinaus ist ebenfalls ein Abstand der maximal das 5-fache des Durchmessers beträgt bevorzugt. In einer weiteren besonders bevorzugten Ausführungsform beträgt der Abstand zwischen den zwei Kontaktöffnungen maximal das 2-fache des Durchmessers der ersten und/oder zweiten Kontaktöffnung.

In einer weiteren bevorzugten Ausführungsform beträgt der Abstand zwischen den Kontaktöffnungen zwischen 6 und 7 mm, bevorzugt 6,58 mm.

In einer besonders bevorzugten Ausführungsform der Chirurgiegeräte-Mehrfachbuchse ist vorgesehen, dass die Mittelachse der Koaxialbuchse und die Mittelachse der ersten Kontaktöffnung einen Abstand von 28-29 mm, insbesondere 28,58 mm, zueinander aufweisen. Eine derartige Beabstandung ermöglicht das Anwenden eines Steckers mit zwei Kontaktstiften, deren Mittelachsen eine Beabstandung von 28 mm bis 29 mm, vorzugsweise von 28,58 mm, aufweisen. Derartige Stecker werden in der Elektrochirurgie oftmals verwendet, so dass diese Ausführungsform insbesondere die Kompatibilität eines Hochfrequenzgenerators, aufweisend eine derartige Chirurgiegeräte-Mehrfachbuchse, erhöht. Insbesondere durch die innere Koaxialbuchsenöffnung sowie der Beabstandung dieser zur ersten Kontaktöffnung wird die Anwendung eines zuvor beschriebenen Steckers ermöglicht.

Eine weitere bevorzugte Ausführungsform der Chirurgiegeräte-Mehrfachbuchse sieht vor, dass die Mittelachse der Koaxialbuchse und die Mittelachse der zweiten Kontaktöffnung einen Abstand von 21-23 mm, insbesondere von 22 mm, zueinander aufweisen. Eine derartige Beabstandung der Koaxialbuchse und der zweiten Kontaktöffnung ermöglicht die Anwendung eines Steckers, der zwei Kontaktstifte mit einer Beabstandung von 21-23 mm, insbesondere von 22 mm, zueinander aufweist. Derartige Stecker mit Kontaktstiften, die die zuvor genannte Beabstandung aufweisen, werden ebenfalls in der Elektrochirurgie oftmals angewendet.

Diese Anordnung der Koaxialbuchse und der zweiten Kontaktöffnung sowie die zuvor beschriebene Anordnung der Koaxialbuchse und der ersten Kontaktöffnung zeigt die Flexibilität der hier vorliegenden Chirurgiegeräte-Mehrfachbuchse. Durch die kompakte Anordnung der Koaxialbuchse sowie der ersten und zweiten Kontaktöffnung wird ermöglicht, dass ein Stecker mit einer Kontaktstiftbeabstandung von 21-23 mm sowie ein Stecker mit einer Kontaktstiftbeabstandung von 28-29 mm sowie ein Koaxialsteckerelement in diese kompakte Chirurgiegeräte-Mehrfachbuchse anschließbar sind. Darüber hinaus wird deutlich, dass bei der zuvor beschriebenen Anordnung keine Mehrfachbelegung von mehr als einem Stecker und somit kein Anschluss von mehr als einem elektrochirurgischen Instrument an die Chirurgiegeräte-Mehrfachbuchse ermöglicht wird. Dadurch ergibt sich eine deutlich bessere Sicherheitssituation, da kein Falschanschluss oder ungewollter Mehrfachanschluss ermöglicht wird.

Gemäß einer weiteren Ausführungsform der Chirurgiegeräte-Mehrfachbuchse ist vorgesehen, dass die Koaxialbuchse ein Anschlussende aufweist, in das ein Koaxialsteckerelement einführbar ist, und/oder die erste Kontaktöffnung ein Anschlussende aufweist, in das ein Kontaktstift einführbar ist, und/oder die zweite Kontaktöffnung ein Anschlussende aufweist, in das ein Kontaktstift einführbar ist, und/oder die Positionierungsöffnung ein Anschlussende aufweist, in das ein Positionierungsstift einführbar ist, wobei mindestens zwei Anschlussenden in einer gemeinsamen Ebene liegen.

Die Anschlussenden der Kontaktöffnungen und/oder der Koaxialbuchse und/oder der Positionierungsöffnung sind insbesondere dahingehend zu verstehen, dass an diesen ein einzuführendes Element eintreten bzw. eingeführt werden kann. In einer gemeinsamen Ebene liegend bedeutet insbesondere, dass die Orthogonalen am Anschlussende von parallelen Mittelachsen der Kontaktöffnungen und/oder Positionierungsöffnung und/oder Koaxialbuchse eine zweidimensionale Geometrie bzw. eine Ebene aufspannen. In einer bevorzugten Variante dieser Ausführungsform ist vorgesehen, dass mindestens drei Anschlussenden in einer gemeinsamen Ebene liegen und alternativ vorzugsweise ist vorgesehen, dass alle Anschlussenden in einer gemeinsamen Ebene liegen. Eine Anordnung von drei oder mehr Anschlussenden in einer gemeinsamen Ebene ist dadurch vorteilhaft, dass eine besonders kompakte Bauweise der Chirurgiegeräte-Mehrfachbuchse erreicht werden kann.

Gemäß einem nicht beanspruchten wird die eingangs genannte Aufgabe gelöst durch eine Chirurgiegeräte-Mehrfachbuchse für einen Hochfrequenzgenerator zum Anschluss eines elektrochirurgischen Instruments, insbesondere eines monopolaren Applikators, umfassend eine Koaxialbuchse, die ausgebildet ist, ein Koaxialsteckerelement aufzunehmen, und eine erste Kontaktöffnung, die ausgebildet ist, einen Kontaktstift aufzunehmen, und eine zweite Kontaktöffnung, die ausgebildet ist, einen Kontaktstift aufzunehmen, und eine dritte Kontaktöffnung, die ausgebildet ist einen Kontaktstift aufzunehmen, wobei vorzugsweise Mittelpunkte der ersten Kontaktöffnung und der zweiten Kontaktöffnung und der dritten Kontaktöffnung auf einer geraden Linie angeordnet sind, und eine vierte Kontaktöffnung.

Insbesondere ist es bevorzugt, dass die erste Kontaktöffnung und die zweite Kontaktöffnung und die dritte Kontaktöffnung derart angeordnet sind, dass ein 3-Pin-Stecker, insbesondere ein 3-Pin-Stecker mit 4 mm Kontaktstift-Durchmesser, mittels der ersten Kontaktöffnung und der zweiten Kontaktöffnung und der dritten Kontaktöffnung an die Chirurgiegeräte-Mehrfachbuchse angeschlossen werden kann, indem die Kontaktstifte des Drei-Pin-Steckers in der ersten Kontaktöffnung, der zweiten Kontaktöffnung und der dritten Kontaktöffnung angeordnet werden. Somit wird die Kompatibilität der Chirurgiegeräte-Mehrfachbuchse weiter gesteigert, indem neben Einzel- und Doppelsteckern, mit einem bzw. zwei Kontaktstiften, sowie dem Koaxialstecker, in dieser Fortbildung ein 3-Pin-Stecker, mit drei Kontaktstiften, anschließbar ist. Ferner ist das Anschließen von Spezialsteckern, beispielsweise zur Wartung, vereinfacht, indem der Wartungsstecker für eine definierte Kontaktöffnungskombination ausgebildet ist.

In einer weiteren bevorzugten Fortbildung der Chirurgiegeräte-Mehrfachbuchse ist vorgesehen, dass die vierte Kontaktöffnung und eine, zwei oder mehrere weitere Kontaktöffnungen jeweils einen zu einer Einsteckrichtung in orthogonaler Richtung ausgerichteten Durchmesser aufweisen, wobei die eine, zwei oder mehreren weiteren Kontaktöffnungen ausgewählt sind aus der Gruppe bestehend aus der ersten Kontaktöffnung, und der zweiten Kontaktöffnung, und der dritten Kontaktöffnung, wobei der Durchmesser der vierten Kontaktöffnung größer ist als der Durchmesser bzw. die Durchmesser der einen, zwei oder mehreren weiteren Kontaktöffnungen, wobei der Durchmesser der vierten Kontaktöffnung vorzugsweise zwischen 6 mm bis 10 mm, insbesondere zwischen 7,5 mm bis 8,5 mm, ausgebildet ist.

Die vierte Kontaktöffnung weist vorzugsweise den größten Durchmesser der angeordneten Kontaktöffnungen, insbesondere im Vergleich zur ersten, zweiten und dritten Kontaktöffnung, auf, wobei die Koaxialbuchse in der Regel einen größeren Durchmesser am äußeren Umfang aufweist. Durch die Anordnung einer vierten Kontaktöffnung besteht die Möglichkeit einer besonderen Kompatibilitätserhöhung. Infolgedessen besteht die Möglichkeit der Anordnung einer Vielzahl derzeit relevanter Stecker im medizintechnischen Bereich, wohingegen im Stand der Technik bekannte Buchsen eine solche Kompatibilität nicht bieten. Dies kann zum einen zu einer Reduktion der Gesamtbuchsenanzahl an einem Hochfrequenz-Generator führen und zum anderen die Kosten reduzieren. Die Ursachen der Kostenreduktion sind vielfältig. Zunächst wird gegebenenfalls eine geringere Gesamtbuchsenanzahl benötigt, womit der Aufwand in der Fertigung und Montage reduziert werden kann. Ferner reduziert sich die Anzahl möglicher Varianten an Hochfrequenz-Generatoren, so dass im Entwicklungsbereich aber auch im Vertrieb die Komplexität reduziert werden kann und somit besser steuerbar ist. Ein Kunde muss sich ferner nicht zwischen mehreren Varianten entscheiden oder die teurere Variante mit einer hohen Gesamtbuchsenanzahl wählen.

Ferner ist es bevorzugt, dass die erste Kontaktöffnung und/oder die zweite Kontaktöffnung und/oder die dritte Kontaktöffnung einen Durchmesser von 4 mm mit einer im Bereich von einschlägigen Steckverbindungen üblichen Toleranz, insbesondere einer Toleranz von plus 0,1 mm und minus 0,1 mm, aufweist. Darüber hinaus kann der Durchmesser der vierten Kontaktöffnung 8 mm betragen, mit einer im Bereich von einschlägigen Steckverbindungen üblichen Toleranz, insbesondere einer Toleranz von plus 0,1 mm und minus 0,1 mm.

Eine bevorzugte Ausführungsvariante der Chirurgiegeräte-Mehrfachbuchse sieht vor, dass die Koaxialbuchse und/oder die vierte Kontaktöffnung zwischen der zweiten Kontaktöffnung und der dritten Kontaktöffnung angeordnet ist bzw. sind. Insbesondere ist bzw. sind die Koaxialbuchse und/oder die vierte Kontaktöffnung in Bezug auf die Richtung einer Verbindungslinie, verbindend die Mittelpunkte der ersten Kontaktöffnung und der zweiten Kontaktöffnung und der dritten Kontaktöffnung, zwischen der zweiten Kontaktöffnung und der dritten Kontaktöffnung angeordnet. Die Anordnung zwischen der zweiten und dritten Kontaktöffnung ermöglicht die Anordnung einer Vielzahl von Steckern, obwohl zur Anordnung der Kontaktöffnungen und der Koaxialbuchse lediglich ein geringer Flächenbedarf besteht.

In einer weiteren bevorzugten Fortbildung der Chirurgiegeräte-Mehrfachbuchse ist vorgesehen, dass die Koaxialbuchse und die vierte Kontaktöffnung derart angeordnet sind, dass eine deren Mittelpunkte verbindende Linie orthogonal zu einer Verbindungslinie, verbindend die Mittelpunkte der ersten Kontaktöffnung und der zweiten Kontaktöffnung und der dritten Kontaktöffnung, ausgerichtet ist. Im Betriebszustand sind die Koaxialbuchse und die vierte Kontaktöffnung vorzugsweise in Bezug auf deren Mittelpunkte vertikal übereinander angeordnet, wobei dann die erste Kontaktöffnung, und die zweite Kontaktöffnung, und die dritte Kontaktöffnung horizontal nebeneinander angeordnet sind, so dass die Verbindungslinie verbindend deren Mittelpunkte ebenfalls horizontal verläuft. Alternativ vorzugsweise sind die Koaxialbuchse und die vierte Kontaktöffnung im Betriebszustand in Bezug auf deren Mittelpunkte horizontal nebeneinander angeordnet, wobei dann die erste Kontaktöffnung, und die zweite Kontaktöffnung, und die dritte Kontaktöffnung vertikal übereinander angeordnet sind, so dass die Verbindungslinie verbindend deren Mittelpunkte ebenfalls horizontal verläuft.

In Folge dieser Anordnung kann der Flächenbedarf am Generator gering gehalten werden mit dem Vorteil, dass der Generator besonders kompakt ausgebildet sein kann und/oder Raum für zusätzliche Funktionen und/oder Funktionselemente, wie beispielsweise Bildschirme oder Bedienelemente, am Generator geschaffen werden kann. Insbesondere kann so auch der Anschluss mehrerer Stecker gleichzeitig ermöglicht oder zumindest vereinfacht werden. Ferner ist vorzugsweise vorgesehen, dass die erste Kontaktöffnung auf der Seite der zweiten Kontaktöffnung angeordnet ist, die der dritten Kontaktöffnung abgewandt ist. Ferner kann es bevorzugt sein, dass die erste Kontaktöffnung auf der Seite der zweiten Kontaktöffnung angeordnet ist, die der dritten Kontaktöffnung abgewandt ist

Eine weitere bevorzugte Ausführungsform sieht vor, dass die Koaxialbuchse und die vierte Kontaktöffnung derart beabstandet zueinander sind, dass in der Koaxialbuchse ein Koaxialstecker und in der vierten Kontaktöffnung ein Kontaktstift, insbesondere ein Boviestecker, zeitgleich angeordnet werden können. Insbesondere ist es bevorzugt, dass die Mittelpunkte der Koaxialbuchse und der vierten Kontaktöffnung mit einer Distanz voneinander beabstandet sind, wobei die Distanz vorzugsweise größer als ein einfacher Durchmesser, oder größer als ein 1,5-facher Durchmesser, oder größer als ein 2-facher Durchmesser, oder größer als ein 2,5-facher Durchmesser, oder größer als ein 3-facher Durchmesser, oder größer als ein 4-facher Durchmesser der vierten Kontaktöffnung ist.

Eine vorteilhafte Ausführungsform sieht ferner vor, dass die vierte Kontaktöffnung in Richtung der geraden Verbindungslinie im montierten Zustand oberhalb der geraden Linie, verbindend die Mittelpunkte der ersten Kontaktöffnung und der zweiten Kontaktöffnung und der dritten Kontaktöffnung, angeordnet ist.

Außerdem kann es bevorzugt sein, dass die Koaxialbuchse in Richtung der geraden Verbindungslinie im montierten Zustand unterhalb der geraden Linie, verbindend die Mittelpunkte der ersten Kontaktöffnung und der zweiten Kontaktöffnung und der dritten Kontaktöffnung, angeordnet ist.

Gemäß einem weiteren Aspekt der Erfindung wird die eingangs genannte Aufgabe gelöst durch einen Elektrochirurgie-Hochfrequenzgenerator, umfassend eine Chirurgiegeräte-Mehrfachbuchse gemäß einer der zuvor beschriebenen Ausführungsformen. Ein derartiger Elektrochirurgie-Hochfrequenzgenerator zeichnet sich zunächst einmal dadurch aus, kompatibel mit einer Vielzahl unterschiedlicher elektrochirurgischer Instrumente zu sein.

Daraus resultiert eine große Auswahl unterschiedlicher Stecker, die mit dem Elektrochirurgie-Hochfrequenzgenerator verwendet werden können. Diese Stecker können sich einerseits hinsichtlich der Beabstandung der vorgesehenen Kontaktstifte unterscheiden, und andererseits darin, dass der Stecker einen Positionierungsstift aufweist oder nicht. Darüber hinaus können Stecker ein Koaxialsteckerelement aufweisen oder nicht. Die große Auswahl an unterschiedlichen Steckern für elektrochirurgische Instrumente resultiert in der Anforderung an Hochfrequenzgeneratoren, dass diese in der Lage sind, auch unterschiedliche Stecker aufzunehmen bzw. mit diesen kompatibel zu sein.

Gemäß einem weiteren Aspekt der vorliegenden Erfindung, wird die eingangs genannte Aufgabe gelöst durch einen Elektrochirurgiegerätestecker zum Einstecken in eine Chirurgiegeräte-Mehrfachbuchse gemäß einer der zuvor beschriebenen Ausführungsformen, umfassend ein Koaxialsteckerelement, und mindestens einen Kontaktstift. Besonders bevorzugt ist ein derartiger Elektrochirurgiegerätestecker, deren Mittelachse des Koaxialsteckerelements und Mittelachse des Kontaktstiftes 22-23 mm voneinander beabstandet sind. Alternativ bevorzugt ist, dass die Mittelachse des Koaxialsteckerelements und die Mittelachse des Kontaktstiftes 28-29 mm voneinander beabstandet sind.

Darüber hinaus ist bevorzugt, dass der Elektrochirurgiegerätestecker einen Positionierungsstift aufweist, welcher ausgebildet und angeordnet ist, in eine Positionierungsöffnung eingeführt zu werden. Ein derartiger Stecker kann verschiedene Nachteile bestehender Steckerarten reduzieren bzw. gar aufheben, wobei ein derartiger Stecker in bisherigen Lösungen nicht angewendet wurde, weil die Anschlussbuchsen in Elektrochirurgie-Hochfrequenzgeneratoren in der Regel nicht in der Lage waren, einen Stecker aufzunehmen, der ein Koaxialsteckerelement und einen Kontaktstift aufweist.

Gemäß einem weiteren Aspekt der vorliegenden Erfindung, wird die eingangs genannte Aufgabe gelöst durch ein elektrochirurgisches System, umfassend einen Hochfrequenzgenerator gemäß der zuvor beschriebenen Ausführungsform, und ein über die Chirurgiegeräte-Mehrfachbuchse des Hochfrequenzgenerators angeschlossenes oder anschließbares elektrochirurgisches Instrument. Ein derartiges System ermöglicht neben einer kompakten Bauweise eine flexible Anwendung unterschiedlicher elektrochirurgischer Instrumente von verschiedenen Herstellern sowie auch elektrochirurgische Instrumente mit unterschiedlichen Steckern. Darüber hinaus ist es bei dem vorliegenden System nicht möglich, eine fehlerhafte Steckverbindung herzustellen. Ferner ist es dem Anwender nicht möglich, mehr als ein elektrochirurgisches Instrument gleichzeitig an den Hochfrequenzgenerator anzuschließen. Dadurch können gegebenenfalls Schäden am Hochfrequenzgenerator und/oder am elektrochirurgischen System vermieden werden. Vorzugsweise weist das an die Chirurgiegeräte-Mehrfachbuchse des Hochfrequenzgenerators angeschlossene oder anschließbare elektrochirurgische Instrument einen zuvor beschriebenen Elektrochirurgiegerätestecker auf.

Gemäß einem weiteren Aspekt der vorliegenden Erfindung wird die eingangs genannte Aufgabe gelöst durch ein Kabel für ein elektrochirurgisches Instrument, dadurch gekennzeichnet, dass das Kabel mit einem Elektrochirurgiegerätestecker nach einem der zuvor erläuterten Aspekte der Erfindung verbunden ist. Das Kabel ist vorzugsweise als mehradriger Verbund von mehreren, voneinander isolierten Einzelleitungen ausgebildet, wobei die die Einzelleitungen insbesondere zur Übertragung von Energie, vorzugsweise eines Stroms, und/oder zur Übermittlung von Informationen bzw. Daten dienen. Darüber hinaus kann das Kabel auch eine weitere Leitung zum Transport von Fluiden, beispielsweise von Kühlflüssigkeiten, beinhalten. Das Kabel ist ferner vorzugsweise mit einem Isoliermaterial ummantelt, wobei das Kabel dennoch in der Regel vorzugsweise elastische Eigenschaften aufweist, so dass das Kabel biegsam ist. Die Verbindung des Kabels mit dem Elektrochirurgiegerätestecker kann, insbesondere für einen Anwender, lösbar, beispielsweise durch eine Steckverbindung, oder unlösbar gestaltet sein.

Gemäß einem weiteren Aspekt der vorliegenden Erfindung wird die eingangs genannte Aufgabe gelöst durch ein elektrochirurgisches Instrument, dadurch gekennzeichnet, dass das elektrochirurgische Instrument mit einem Kabel gemäß dem zuvor beschriebenen Aspekt verbunden ist. Eine Verbindung an einer Verbindungsstelle zwischen dem Kabel und dem elektrochirurgischen Instrument kann, insbesondere für einen Anwender, lösbar oder unlösbar gestaltet sein. Eine lösbare Verbindung zwischen Kabel und elektrochirurgischen Instrument hat den Vorteil, dass im Falle eines Defekts des Kabels oder des Instruments lediglich die defekte Komponente ausgetauscht werden muss und die intakte Komponente weiterhin verwendet werden kann.

Unter einer unlösbaren Verbindung wird eine Verbindung verstanden, die nicht dafür vorgesehen ist von einem Anwender gelöst zu werden. Eine, insbesondere für den Anwender, unlösbare Verbindung hat wiederum den Vorteil, dass ein unbeabsichtigtes Trennen der Verbindung, insbesondere während der Anwendung des elektrochirurgischen Instruments, verhindert oder zumindest erschwert wird, so dass die Sicherheit während der Verwendung, insbesondere während einer chirurgischen Operation, gesteigert wird.

Für weitere Vorteile, Ausführungsformen und Ausführungsdetails dieser weiteren Aspekte und ihrer möglichen Fortbildungen wird auch auf die zuvor erfolgte Beschreibung zu den entsprechenden Merkmalen und Fortbildungen der Chirurgiegeräte-Mehrfachbuchse verwiesen.

Bevorzugte Ausführungsformen der Erfindung werden beispielhaft anhand der beiliegenden Figuren erläutert. Es zeigen:
- Fig. 1a-1d:: ein Ausführungsbeispiel einer Chirurgiegeräte-Mehrfachbuchse;
- Fig. 2:: einen Hochfrequenzgenerator mit einer beispielhaften Chirurgiegeräte-Mehrfachbuchse;
- Fig. 3:: ein elektrochirurgisches System mit einem Hochfrequenzgenerator und einer beispielhaften Chirurgiegeräte-Mehrfachbuchse;
- Fig. 4:: einen Elektrochirurgiegerätestecker; und
- Fig. 5:: einen Stecker mit zwei Kontaktstiften.
- Fig. 6:: eine im Stand der Technik bekannte Chirurgiegeräte-Mehrfachbuchse für einen Bovie-Stecker;
- Fig. 7:: eine im Stand der Technik bekannte Chirurgiegeräte-Mehrfachbuchse für einen Koaxialstecker;
- Fig. 8a-8d:: ein weiteres Ausführungsbeispiel einer Chirurgiegeräte-Mehrfachbuchse;
- Fig. 9:: eine schematische, dreidimensionale Ansicht der in den Fig. 8a-8d gezeigten Chirurgiegeräte-Mehrfachbuchse mit einem Drei-Pin-Stecker;
- Fig. 10:: eine schematische, dreidimensionale Ansicht der in den Fig. 8a-8d gezeigten Chirurgiegeräte-Mehrfachbuchse mit einem Bovie-Stecker;
- Fig. 11:: eine schematische, dreidimensionale Ansicht der in den Fig. 8a-8d gezeigten Chirurgiegeräte-Mehrfachbuchse mit einem Koaxialstecker;
- Fig. 12:: ein im Stand der Technik bekannter Drei-Pin-Stecker;
- Fig. 13:: ein im Stand der Technik bekannter Stecker mit großem Kontaktstift;

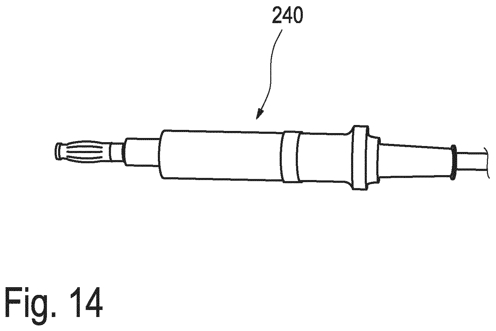

- Fig. 14:: ein im Stand der Technik bekannter Koaxialstecker.

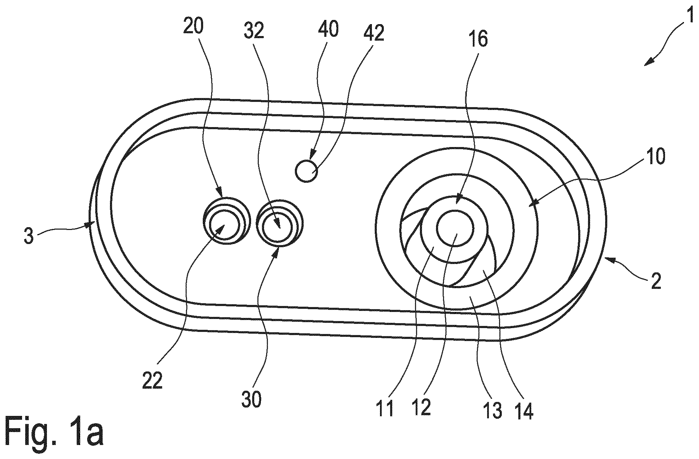

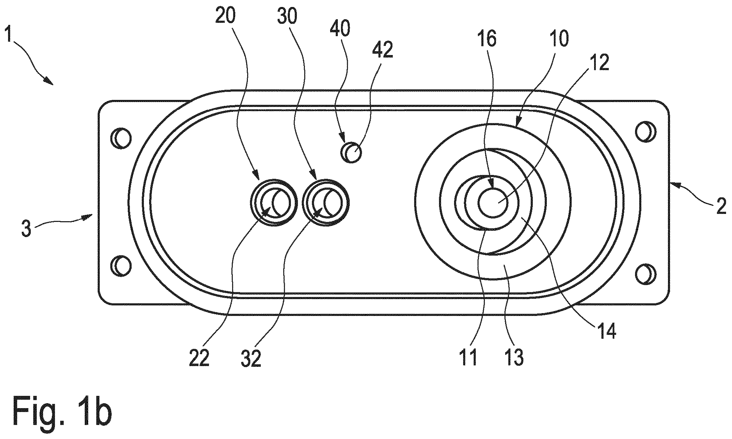

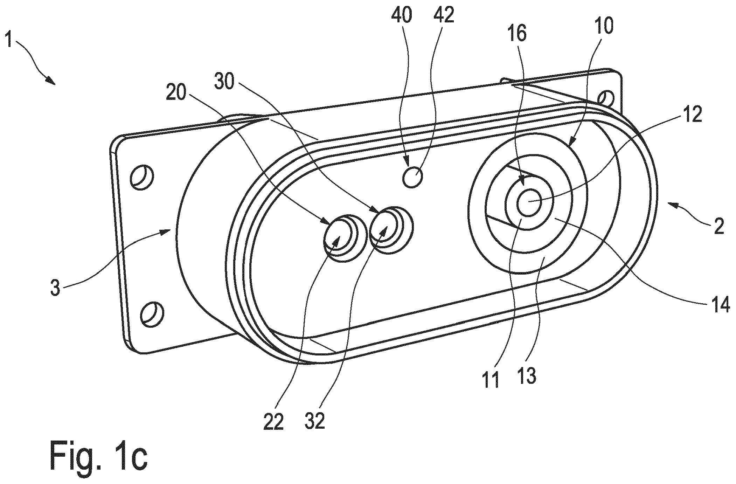

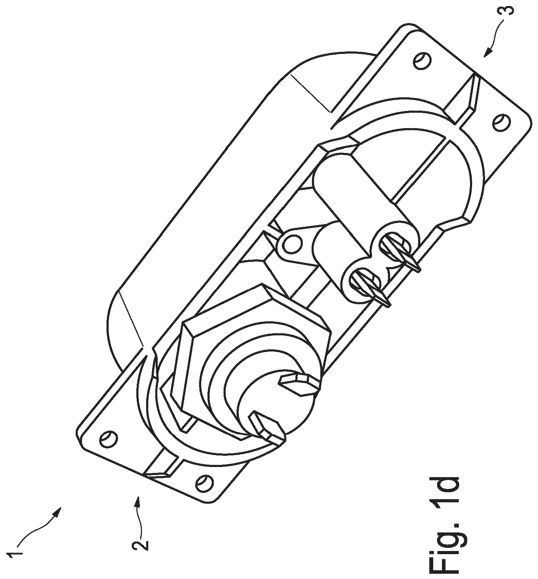

Die Figuren 1a-1d zeigen ein Ausführungsbeispiel einer Chirurgiegeräte-Mehrfachbuchse 1. Die Chirurgiegeräte-Mehrfachbuchse 1 erstreckt sich von einem ersten Ende 2 hin zu einem zweiten Ende 3. Die Chirurgiegeräte-Mehrfachbuchse 1 weist eine flachovale Außenkontur auf, die zwei parallele, gegenüberliegende Flanken aufweist und am ersten und zweiten Ende 2, 3 eine gekrümmte, insbesondere halbkreisförmige Geometrie aufweist. In einem Abschnitt angrenzend an das erste Ende 2 der Chirurgiegeräte-Mehrfachbuchse 1 ist eine Koaxialbuchse 10 angeordnet.

Die Koaxialbuchse 10 wird gebildet durch zwei koaxial angeordnete Hülsen. Die innere Hülse 11 wird von der äußeren Hülse 13 in radialer Richtung im Wesentlichen umschlossen. Ferner weist die innere Hülse 11 einen Außendurchmesser auf, der eine geringere Abmessung aufweist als der Innendurchmesser der äußeren Hülse 13. Dadurch werden zwei Öffnungen gebildet, die durch den Zwischenraum zwischen der äußeren und der inneren Hülse 13, 11 und den geometriebedingten Hohlraum der inneren Hülse 11 gebildet werden. Der Zwischenraum zwischen der äußeren und der inneren Hülse 13, 11 bildet die äußere, ringförmige Koaxialbuchsenöffnung 14 und der im Inneren der inneren Hülse 11 gebildete Raum bildet die innere Koaxialbuchsenöffnung 12.

In einem Abschnitt, der an das zweite Ende 3 der Chirurgiegeräte-Mehrfachbuchse 1 angrenzt, sind die erste Kontaktöffnung 20 und die zweite Kontaktöffnung 30 angeordnet. Die Kontaktöffnungen 20, 30 weisen einen orthogonal zur Mittelachse kreisrunden Querschnitt auf, der jeweils im Wesentlichen den gleichen Durchmesser wie die innere Koaxialbuchsenöffnung 12 hat. Die erste Kontaktöffnung 20 und die zweite Kontaktöffnung 30 liegen auf einer gedanklichen Verbindungslinie, die von dem ersten Ende 2 zum zweiten Ende 3 verläuft. Dabei ist die erste Kontaktöffnung 20 dem zweiten Ende 3 zugewandt und die zweite Kontaktöffnung 30 ist dem ersten Ende 2 bzw. der Koaxialbuchse 10 zugewandt. Die gedankliche Verbindungslinie, die das erste Ende 2 und das zweite Ende 3 miteinander verbindet, teilt die Chirurgiegeräte-Mehrfachbuchse 1 gedanklich in zwei, horizontal getrennte Hälften, die in vertikaler Richtung jeweils die gleiche Abmessung aufweisen. Auf dieser Linie liegen die Mittelpunkte der ersten Kontaktöffnung 20, der zweiten Kontaktöffnung 30 sowie der Koaxialbuchse 10.

Die Chirurgiegeräte-Mehrfachbuchse 1 weist ferner eine Positionierungsöffnung 40 auf. Die Positionierungsöffnung 40 weist orthogonal zur Mittelachse einen kreisrunden Querschnitt auf. Der Durchmesser ist geringer als der Durchmesser der Kontaktöffnungen 20, 30. Ferner liegt die Positionierungsöffnung 40 nicht auf der Verbindungslinie, die die Mittelpunkte der Kontaktöffnungen 30, 40 und der inneren Koaxialbuchse 10 verbindet, so dass die Positionierungsöffnung 40 auch nicht in einer Flucht mit der ersten und zweiten Kontaktöffnung 20, 30 sowie der Koaxialbuchse 10 liegt.

Die Koaxialbuchse 10, die Kontaktöffnungen 20, 30 und die Positionierungsöffnung 40 sind im Wesentlichen in einer ebenen Fläche der Chirurgiegeräte-Mehrfachbuchse 1 eingelassen. Dabei sind ein Anschlussende 22 der ersten Kontaktöffnung 20 und ein Anschlussende 32 der zweiten Kontaktöffnung 30 leicht in diese Ebene hineinversetzt, so dass diese zurückgesetzt sind. Die Anschlussöffnung 42 der Positionierungsöffnung 40 und das Anschlussende 16 der Koaxialbuchse 10 liegen im Wesentlichen in einer Ebene mit der zuvor bezeichneten Ebene der Chirurgiegeräte-Mehrfachbuchse 1.

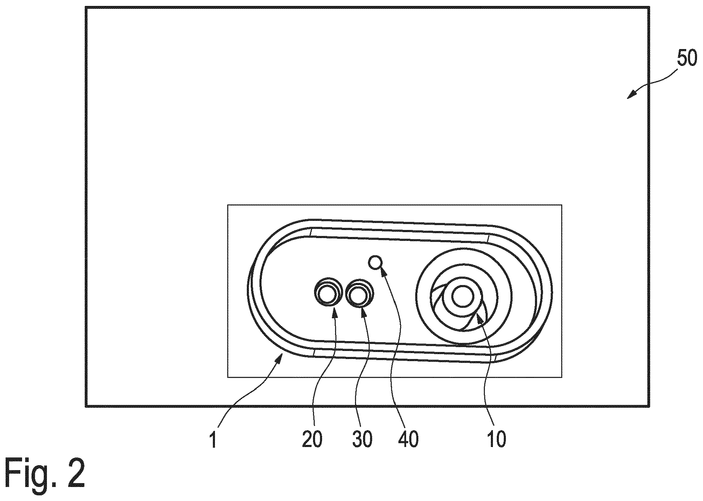

Figur 2 zeigt einen Hochfrequenzgenerator 50 mit einer Chirurgiegeräte-Mehrfachbuchse 1. Die Chirurgiegeräte-Mehrfachbuchse 1 dieses Hochfrequenzgenerators 50 weist ebenfalls die zuvor bezeichnete Koaxialbuchse 10, die erste Kontaktöffnung 20 und die zweite Kontaktöffnung 30 auf. Darüber hinaus weist die Chirurgiegeräte-Mehrfachbuchse 1 die Positionierungsöffnung 40 auf.

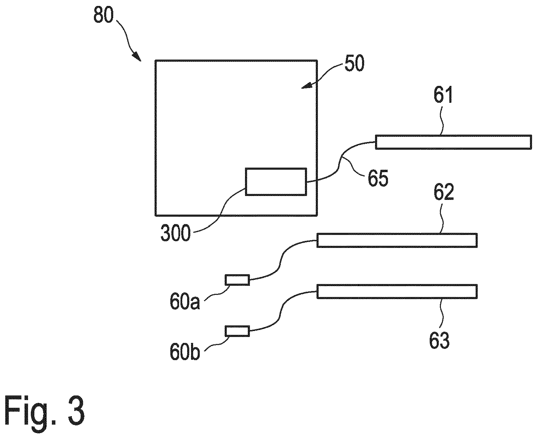

Figur 3 zeigt ein elektrochirurgisches System 80 mit einem Hochfrequenzgenerator 50 und einer erfindungsgemäßen Chirurgiegeräte-Mehrfachbuchse 300, an die ein erstes elektrochirurgisches Instrument 61 angeschlossen ist. Das elektrochirurgische System 80 umfasst den Hochfrequenzgenerator 50 mit einer weiteren Ausführungsform der Chirurgiegeräte-Mehrfachbuchse 300 sowie ein erstes elektrochirurgisches Instrument 61. Das elektrochirurgische Instrument 61 ist mit einem Verbindungskabel 65 und über einen daran angeordneten Stecker (nicht dargestellt) an die Chirurgiegeräte-Mehrfachbuchse 300 angeschlossen. Die Chirurgiegeräte-Mehrfachbuchse 300 ist an dem Hochfrequenzgenerator 50 angeordnet. Darüber hinaus stehen ein zweites elektrochirurgisches Instrument 62 und ein drittes elektrochirurgisches Instrument 63 bereit. Die elektrochirurgischen Instrumente 62, 63 weisen jeweils einen Stecker 60a, 60b auf, wobei die Stecker 60a, 60b vorzugsweise andersartig sind, beispielsweise ist der Abstand der Kontaktstifte unterschiedlich.

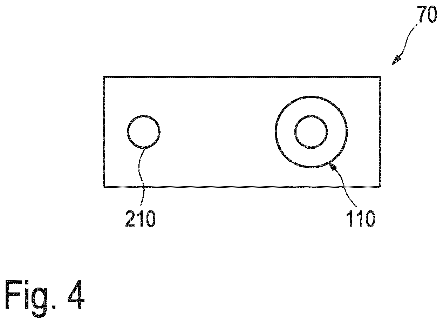

Es ist ferner ein viertes elektrochirurgisches Instrument 64 dargestellt, welches über ein weiteres Verbindungskabel 65a mit einem Elektrochirurgiegerätestecker 70 verbunden ist. Der Elektrochirurgiegerätestecker 70 ist in Figur 4 näher dargestellt. Die Verbindung des vierten elektrochirurgischen Instruments 64 mit dem Verbindungskabel 65a kann als feste Verbindung oder als nicht dargestellte Steckverbindung ausgeführt sein, so dass das Kabel 65a und das elektrochirurgische Instrument 64 vorzugsweise als separate Baugruppen vorgesehen sind.

Figur 4 zeigt einen Elektrochirurgiegerätestecker 70. Der Elektrochirurgiegerätestecker 70 umfasst einen Kontaktstift 210 sowie ein Koaxialsteckerelement 110. Das Koaxialsteckerelement 110 weist dabei eine kreisförmige Hülse auf, wobei diese Hülse dünnwandig gestaltet ist. Innerhalb dieser Hülse ist koaxial ein Kontaktstift angeordnet, der wiederum einen kreisrunden Querschnitt aufweist.

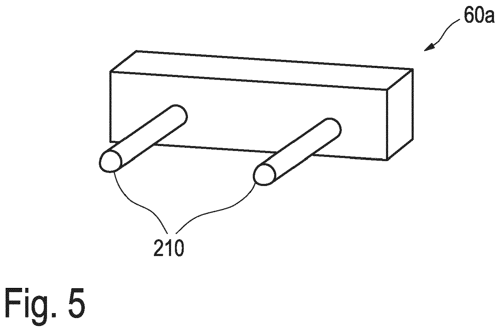

Figur 5 zeigt einen Stecker mit zwei Kontaktstiften 210. Die Figur 5 zeigt schematisch einen handelsüblichen Stecker 60a für ein elektrochirurgisches Instrument 62 bzw. zur Verbindung eines elektrochirurgischen Instruments 62 mit einem Hochfrequenzgenerator 50 über eine Chirurgiegeräte-Mehrfachbuchse. Der Stecker 60a weist an einem Grundelement angeordnet zwei Kontaktstifte 210 auf. Die Kontaktstifte 210 weisen jeweils eine longitudinale Erstreckung auf, wobei orthogonal zur longitudinalen Erstreckung ein kreisrunder Querschnitt ausgebildet wird.

Ein elektrochirurgisches Instrument mit einem Stecker 60a gemäß Figur 5 ist geeignet an den Hochfrequenzgenerator 50 aus Figur 2 angeschlossen zu werden. Dabei besteht die Möglichkeit, zwei unterschiedliche Stecker mit Kontaktstiften 210 einzusetzen. Zum einen besteht die Möglichkeit einen Stecker zu verwenden, dessen einer Kontaktstift 210 in die innere Koaxialbuchsenöffnung 12 und dessen anderer Kontaktstift 210 in die erste Kontaktöffnung 20 eingeführt wird. Darüber hinaus besteht die Möglichkeit, einen Stecker zu verwenden, dessen erster Kontaktstift 210 ebenfalls in die innere Koaxialbuchsenöffnung 12 eingeführt wird, wobei jedoch der zweite Kontaktstift 210 in die zweite Kontaktöffnung 30 der Chirurgiegeräte-Mehrfachbuchse 1, 300 eingeführt wird.

Das zuvor genannte Einführen der Kontaktstifte 210 in die Öffnungen geschieht vorzugsweise gleichzeitig, da die Kontaktstifte 210 fest und in einer Ebene liegend an einem Grundkörper des Steckers 60a angeordnet sind. Dadurch dass die Chirurgiegeräte-Mehrfachbuchse 1, 300 die erste und zweite Kontaktöffnung umfasst, wird die Möglichkeit gegeben, Stecker einzusetzen, deren Abstand zwischen den Kontaktstiften 210 unterschiedlich ist. Darüber hinaus besteht die Möglichkeit einen Stecker zu verwenden der für eine Koaxialbuchse geeignet ist, beispielsweise ein Koaxialsteckerelement 110.

Darüber hinaus ist es möglich, neben diesen am Markt verfügbaren Steckern einen speziellen Elektrochirurgiegerätestecker 70 zu verwenden, der ein Koaxialsteckerelement 110 und mindestens einen Kontaktstift 210 umfasst. Ferner besteht die Möglichkeit, einen Stecker zu verwenden, der ein Koaxialsteckerelement 110 und zwei Kontaktstifte 210 umfasst, wobei in diesem Fall die Beabstandung der zwei Kontaktstifte 210 die gleiche Abmessung aufzuweisen hat, wie die Beabstandung der zwei Kontaktöffnungen 20, 30. Darüber hinaus besteht die Möglichkeit, dass ein Stecker verwendet wird, der eine der zuvor genannten Ausführungsformen umfasst, jedoch zusätzlich noch einen Positionierungsstift umfasst, wobei dieser Positionierungsstift in die Positionierungsöffnung 40 der Chirurgiegeräte-Mehrfachbuchse 1, 300 eingeführt wird.

Dadurch dass unterschiedliche Stecker, insbesondere unterschiedlich hinsichtlich der Anzahl der Kontaktstifte 210, des Koaxialsteckerelements 110 und des Positionierungsstifts (nicht dargestellt), in die Chirurgiegeräte-Mehrfachbuchse 1, 300 eingeführt werden können, besteht für den Anwender nun nicht mehr die Gefahr, dass er einen Stecker in die falsche Buchse am Hochfrequenzgenerator einführt und/oder ungewollt inkompatible Geräte gleichzeitig mit dem Hochfrequenzgenerator verbindet oder betreibt. Ein derartiges Vertauschen bzw. Einführen eines Steckers in eine falsche Buchse kann zu Schäden am Gerät oder am Patienten insbesondere während des Chirurgieprozesses führen, so dass durch die erfindungsgemäße Lösung die Sicherheit während des Chirurgieprozesses optimiert wird.

Darüber hinaus resultiert die hohe Anzahl kompatibler Stecker darin, dass lediglich eine Chirurgiegeräte-Mehrfachbuchse in den Hochfrequenzgenerator 50 integriert werden muss und nicht wie zuvor für jeden Stecker eine spezifisch auf diesen Stecker ausgelegte Buchse. Dadurch wiederum resultiert ein geringerer Flächenbedarf für die Chirurgiegeräte-Mehrfachbuchse, so dass eine kompaktere Bauweise des Hochfrequenzgenerators realisiert werden kann. Des Weiteren können elektrochirurgische Instrumente von unterschiedlichen Herstellern und mit unterschiedlichen Steckern an einem einzelnen Hochfrequenzgenerator angewendet werden, so dass in kostengünstiger Weise nur noch ein einzelner Hochfrequenzgenerator verwendet werden muss, der eine Vielzahl an unterschiedlichen elektrochirurgischen Instrumenten bedienen kann, ohne dass hierzu Adapter verwendet werden müssen.

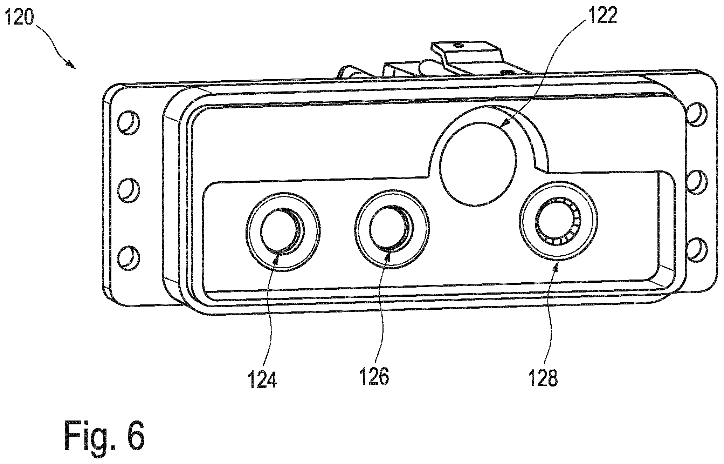

Figur 6 zeigt eine im Stand der Technik bekannte Chirurgiegeräte-Mehrfachbuchse für einen Bovie-Stecker. Die Chirurgiegeräte-Mehrfachbuchse 120 weist eine erste Kontaktöffnung 124, eine zweite Kontaktöffnung 126 und eine dritte Kontaktöffnung 128 auf. Die zweite Kontaktöffnung 126 ist benachbart zur ersten Kontaktöffnung 124 angeordnet, und die dritte Kontaktöffnung 128 ist benachbart zu der zweiten Kontaktöffnung 126 angeordnet, wobei die erste Kontaktöffnung 124 nicht benachbart zur dritten Kontaktöffnung 128 angeordnet ist. Ferner sind die Kontaktöffnungen 124, 126, 128 derart auf einer geraden Linie angeordnet, dass die Mittelpunkte der Kontaktöffnungen 124, 126, 128 auf der geraden Linie angeordnet sind. Eine Anordnung mehrerer Stecker ist bei der Chirurgiegeräte-Mehrfachbuchse 120 nicht oder lediglich eingeschränkt möglich. Ferner ist die Chirurgiegeräte-Mehrfachbuchse 120 nicht für den Anschluss von Koaxialsteckern ausgebildet.

Ferner weist die Chirurgiegeräte-Mehrfachbuchse 120 eine große Kontaktöffnung 122 auf, die einen Durchmesser in orthogonaler Richtung zur Einsteckrichtung aufweist, der doppelt so groß ist wie der Durchmesser der Kontaktöffnung 124, 126, 128. Ferner ist die große Kontaktöffnung 122 in Bezug auf eine Richtung der zuvor genannten geraden Linie zwischen der zweiten Kontaktöffnung 126 und der dritten Kontaktöffnung 128 angeordnet. Es ist ferner zu erkennen, dass der Mittelpunkt der großen Kontaktöffnung 122 beabstandet von der geraden Linie ist. Im eingebauten Zustand der Chirurgiegeräte-Mehrfachbuchse 120 ist der Mittelpunkt der großen Kontaktöffnung 122 oberhalb der Mittelpunkte der Kontaktöffnung 124, 126, 128 angeordnet. Insbesondere ist die große Kontaktöffnung 122 zum Anschluss von Boviesteckern ausgebildet.

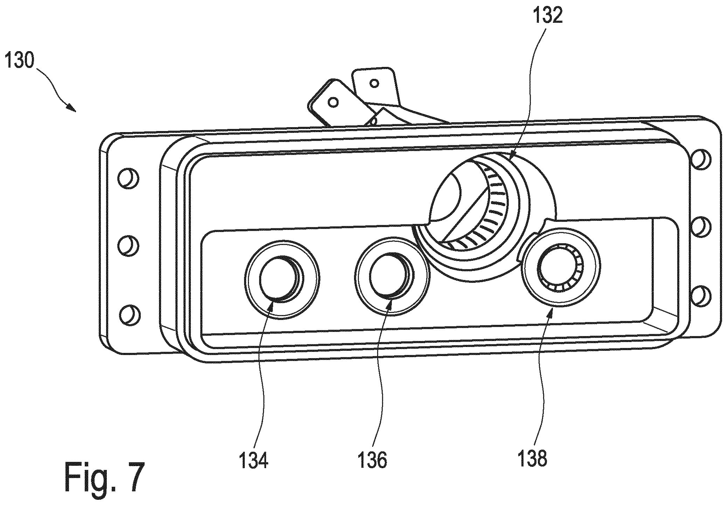

Die in Figur 7 gezeigte, im Stand der Technik bekannte Chirurgiegeräte-Mehrfachbuchse 130 weist im Wesentlichen die gleiche Struktur bzw. Anordnung auf wie die Chirurgiegeräte-Mehrfachbuchse 120 in Figur 6. Die Chirurgiegeräte-Mehrfachbuchse 130 weist eine erste Kontaktöffnung 134, eine zweite Kontaktöffnung 136 und eine dritte Kontaktöffnung 138 auf, die analog zu den Kontaktöffnungen 124, 126, 128 der Chirurgiegeräte-Mehrfachbuchse 120 angeordnet sind. Anstatt der großen Kontaktöffnung 122 der Chirurgiegeräte-Mehrfachbuchse 120 weist die hier gezeigte Chirurgiegeräte-Mehrfachbuchse 130 eine Koaxialbuchse 132 auf. Der Mittelpunkt der Koaxialbuchse 132 ist im Wesentlichen gleich verortet wie der Mittelpunkt der großen Kontaktöffnung 122 an der Chirurgiegeräte-Mehrfachbuchse 120. Auch bei der Chirurgiegeräte-Mehrfachbuchse 130 ist eine Anordnung mehrerer Stecker nicht oder lediglich eingeschränkt möglich. Ferner ist die Chirurgiegeräte-Mehrfachbuchse 130 nicht für den Anschluss von Boviesteckern ausgebildet.

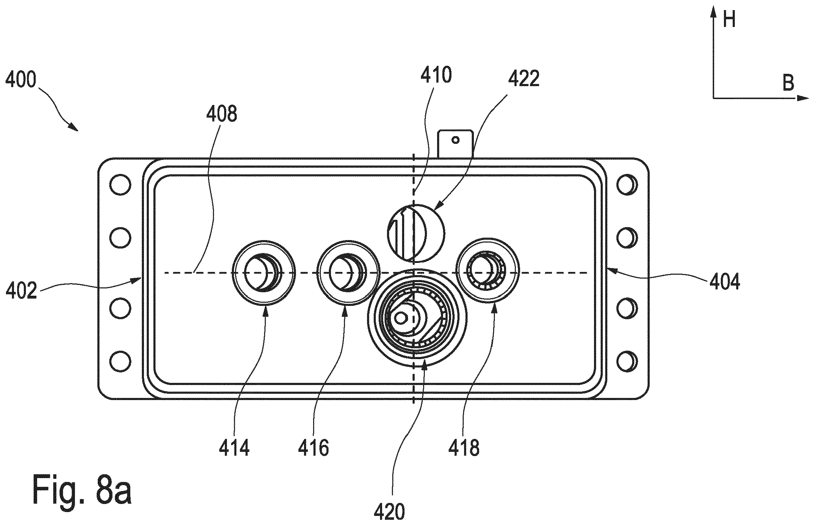

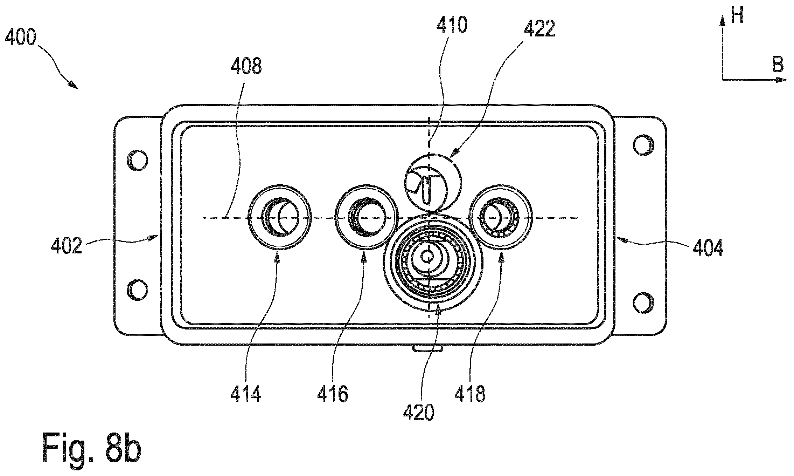

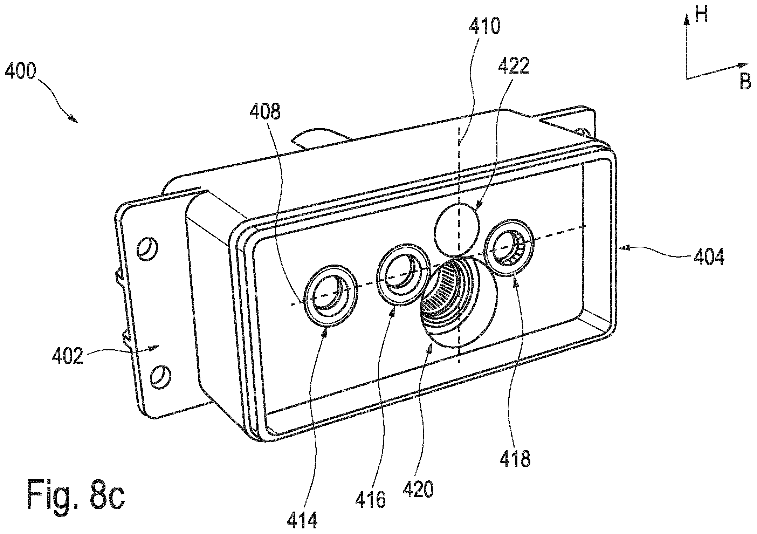

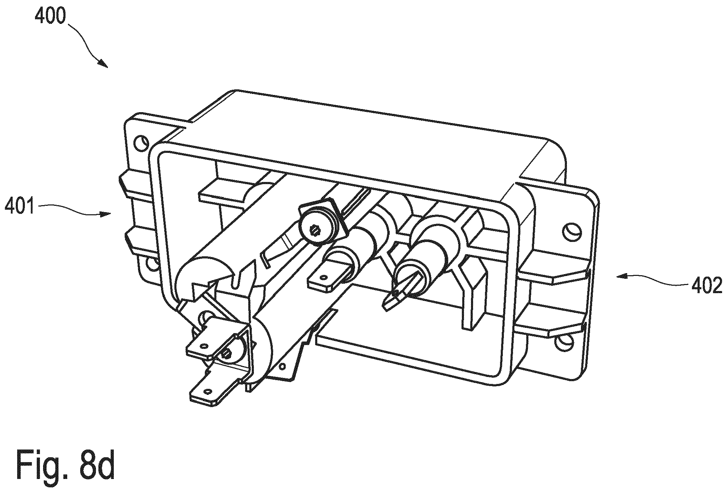

Die Figuren 8a-8d zeigen ein weiteres Ausführungsbeispiel einer erfindungsgemäßen Chirurgiegeräte-Mehrfachbuchse. Die Chirurgiegeräte-Mehrfachbuchse 400 erstreckt sich in Richtung einer Breite B von einem ersten Ende 402 zu einem zweiten Ende 404. Ferner weist die Chirurgiegeräte-Mehrfachbuchse 400 eine erste Kontaktöffnung 414, eine zweite Kontaktöffnung 416 und eine dritte Kontaktöffnung 418 auf. Die erste Kontaktöffnung 414 ist von den genannten Kontaktöffnungen 414, 416, 418 diejenige, die dem ersten Ende zugewandt ist. Die dritte Kontaktöffnung 418 hingegen ist die Kontaktöffnung, die dem zweiten Ende 404 zugewandt ist. Die zweite Kontaktöffnung 416 ist in Richtung der Breite zwischen der ersten Kontaktöffnung 414 und der dritten Kontaktöffnung 418 angeordnet.

Die Kontaktöffnungen 414, 416, 418 weisen jeweils einen gleichen Durchmesser orthogonal zu einer Einsteckrichtung auf und ferner jeweils einen Mittelpunkt auf. Die Mittelpunkte der Kontaktöffnungen 414, 416, 418 liegen auf einer Horizontallinie 408, die in Richtung der Breite B ausgerichtet ist. In Bezug auf die Breite B ist zu erkennen, dass der Abstand zwischen der ersten Kontaktöffnung 414 und der zweiten Kontaktöffnung 416 deutlich geringer ist als der Abstand zwischen der zweiten Kontaktöffnung 416 und der dritten Kontaktöffnung 418.

Die Chirurgiegeräte-Mehrfachbuchse 400 weist ferner eine Koaxialbuchse 420 und eine vierte Kontaktöffnung 422 auf. Die vierte Kontaktöffnung 422 weist einen Durchmesser orthogonal zu einer Einsteckrichtung auf, der die doppelte Abmessung von den jeweiligen Durchmessern der Kontaktöffnungen 414, 416, 418 aufweist. Die Koaxialbuchse 420 und die vierte Kontaktöffnung 422 weisen jeweils einen Mittelpunkt auf, wobei die Mittelpunkte der Koaxialbuchse 420 und der vierten Kontaktöffnung 422 auf einer geraden Vertikallinie 410 angeordnet sind, wobei diese Vertikallinie parallel zu einer Höhe der Chirurgiegeräte-Mehrfachbuchse angeordnet ist. Es ist ferner zu erkennen, dass die Mittelpunkte der Koaxialbuchse 420 und der vierten Kontaktöffnung 422 in Bezug auf die Breite B zwischen der zweiten Kontaktöffnung 416 und der dritten Kontaktöffnung 418 angeordnet sind. Insbesondere ist der Abstand der Mittelpunkte der Koaxialbuchse 420 und der vierten Kontaktöffnung 422 zu dem Mittelpunkt der zweiten Kontaktöffnung 416 und dem Mittelpunkt der dritten Kontaktöffnung 418 gleich. Der Mittelpunkt der Koaxialbuchse 420 liegt in Bezug auf die Höhe unter der Horizontallinie 408. Der Mittelpunkt der vierten Kontaktöffnung 422 liegt in Bezug auf die Höhe über der Horizontallinie 408.

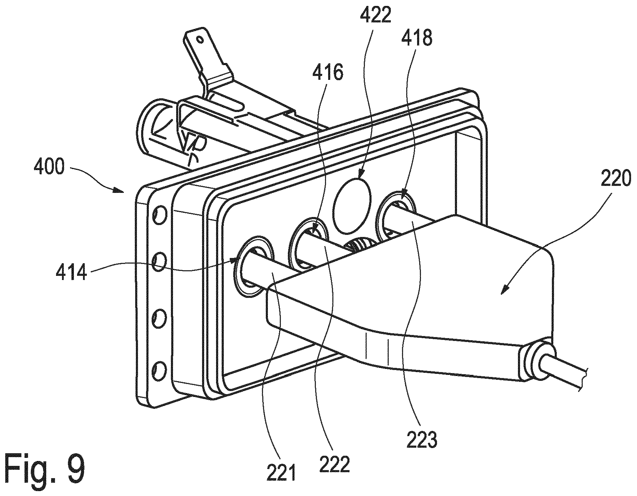

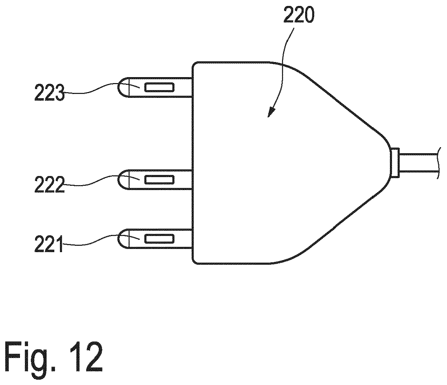

Die Figuren 9 bis 11 zeigen den Einsatz der Chirurgiegeräte-Mehrfachbuchse 400, wobei jeweils ein Zustand mit unterschiedlichen Steckern 220, 230, 240 gezeigt ist. In Figur 9 sind die erste Kontaktöffnung 414, die zweite Kontaktöffnung 416 und die dritte Kontaktöffnung 418 genutzt, um einen Drei-Pin-Stecker 220 mit einem ersten Kontaktstift 221, einem zweiten Kontaktstift 222 und einem dritten Kontaktstift 223 aufzunehmen. Der Drei-Pin-Stecker 220 mit dem ersten Kontaktstift 221, dem zweiten Kontaktstift 222 und dem dritten Kontaktstift 223 ist in anderer Perspektive in Figur 12 gezeigt.

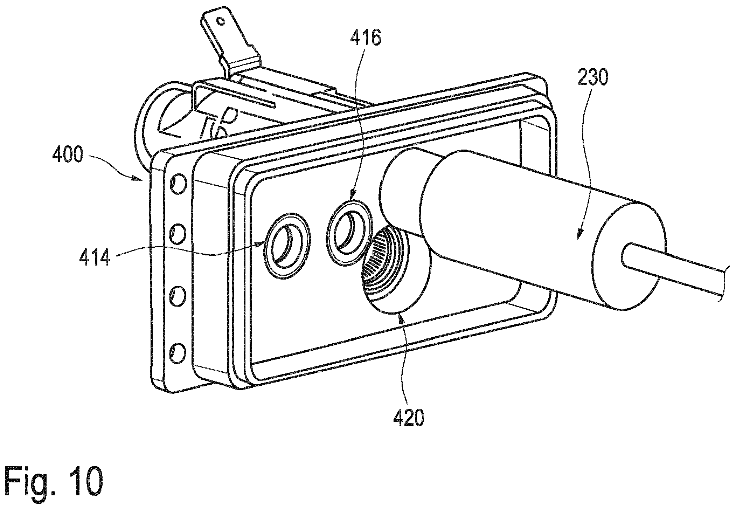

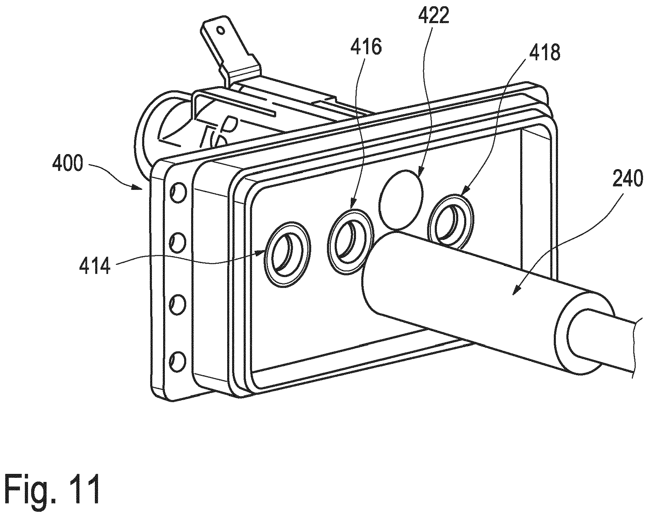

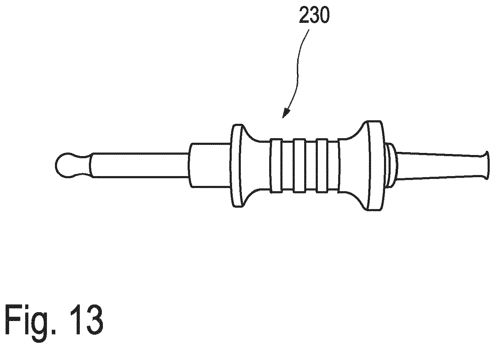

In Figur 10 ist gezeigt, wie an der Chirurgiegeräte-Mehrfachbuchse 400 ein Stecker mit großem Kontaktstift angeordnet ist, der im Stand der Technik auch als Bovie-Stecker bekannt ist. Der Stecker mit großem Kontaktstift 230 ist in anderer Perspektive auch nochmals in Figur 13 gezeigt. Figur 11 zeigt den Einsatz eines Koaxialsteckers an der Chirurgiegeräte-Mehrfachbuchse 400, indem der Koaxialstecker 240 in der Koaxialbuchse 420 angeordnet ist. Ein derartiger Koaxialstecker ist beispielhaft in Figur 13 aus anderer Perspektive gezeigt.

Die Chirurgiegeräte-Mehrfachbuchse 400 weist gegenüber den im Stand der Technik bekannten Chirurgiegeräte-Mehrfachbuchsen eine Vielzahl an Vorteilen auf. Die Steckmöglichkeiten der derzeit verwendeten Buchsen an Chirurgiegeräten werden in einer einzelnen neuen Buchse in bisher nicht bekannter Anordnung integriert, so dass mittels der Chirurgiegeräte-Mehrfachbuchse 400 insgesamt ein praktikablerer, sicherer und kostengünstiger Hochfrequenz-Generator zur Verfügung gestellt werden kann. Die Buchsen können beispielsweise derart angeordnet werden, dass zwei oder mehr Stecker gleichzeitig an der Buchse angeordnet werden können. Somit lassen sich zwei oder mehr Stecker an der Chirurgiegeräte-Mehrfachbuchse 400 anschließen. Daraus folgt, dass für einen Hochfrequenzgenerator lediglich eine Buchse oder gegebenenfalls bei Bedarf zwei Buchsen angeordnet werden müssen, sodass die Anzahl der Buchsen reduziert ist. Daraus resultieren verschiedene Vorteile in Bezug auf die Fertigung der Buchsen an sich und somit für den Hersteller von Hochfrequenzgeneratoren deutlich geringere Kosten. Ferner folgt aus der Anordnung, dass die mehreren Anschlussmöglichkeiten auf einer kompakten Fläche anordenbar sind, so dass insgesamt Fläche am Hochfrequenz-Generator eingespart wird und z.B. eine besonders kompakte Bauform des Generators ermöglicht wird. Da insbesondere der Platz im medizinischen Umfeld teuer ist, können hiermit zusätzlich Kosten reduziert werden. Der eingesparte Platz am Hochfrequenz-Generator kann z.B. auch zur Anordnung weiterer Funktionselemente, wie beispielsweise Bildschirme oder Bedienelemente, genutzt werden. Darüber hinaus resultiert die derartige Anordnung in der Möglichkeit, Falschsteckungen zu vermeiden. Infolgedessen reduziert sich die Wahrscheinlichkeit von Fehlern bei einem chirurgischen Eingriff, sodass die Sicherheit der Anwendung eines elektrochirurgischen Systems mit einer Chirurgiegeräte-Mehrfachbuchse 400 deutlich erhöht wird. Ferner werden durch logistische Vorteile und höhere Einkaufsmengen Vorteile insbesondere im Bereich der Kosten erzielt.

### Bezugszeichen

- 1, 300, 400: Chirurgiegeräte-Mehrfachbuchse
- 2: erstes Ende
- 3: zweites Ende
- 10: Koaxialbuchse
- 11: innere Hülse
- 12: innere Koaxialbuchsenöffnung
- 13: äußere Hülse
- 14: äußere Koaxialbuchsenöffnung
- 16: Anschlussende Koaxialbuchse
- 20: erste Kontaktöffnung
- 22: Anschlussende erste Kontaktöffnung
- 30: zweite Kontaktöffnung
- 32: Anschlussende zweite Kontaktöffnung
- 40: Positionierungsöffnung
- 42: Anschlussende Positionierungsöffnung
- 50: Hochfrequenzgenerator
- 60a, 60b: Stecker
- 61: erstes elektrochirurgisches Instrument
- 62: zweites elektrochirurgisches Instrument
- 63: drittes elektrochirurgisches Instrument
- 64: viertes elektrochirurgisches Instrument
- 65,65a: Verbindungskabel
- 70: Elektrochirurgiegerätestecker
- 80: Elektrochirurgisches System
- 110: Koaxialsteckerelement
- 120: Chirurgiegeräte-Mehrfachbuchse
- 122: große Kontaktöffnung
- 124: erste Kontaktöffnung
- 126: zweite Kontaktöffnung
- 128: dritte Kontaktöffnung
- 130: Chirurgiegeräte-Mehrfachbuchse
- 132: Koaxialbuchse
- 134: erste Kontaktöffnung
- 136: zweite Kontaktöffnung
- 138: dritte Kontaktöffnung
- 210: Kontaktstift
- 220: Drei-Pin-Stecker
- 221: erster Kontaktstift
- 222: zweiter Kontaktstift
- 223: dritter Kontaktstift
- 230: Stecker mit großem Kontaktstift
- 240: Koaxialstecker
- 402: erstes Ende
- 404: zweites Ende
- 408: Horizontallinie
- 410: Vertikallinie
- 414: erste Kontaktöffnung
- 416: zweite Kontaktöffnung
- 418: dritte Kontaktöffnung
- 420: Koaxialbuchse
- 422: vierte Kontaktöffnung
- B: Breite
- H: Höhe

## Patentansprüche

1. Chirurgiegeräte-Mehrfachbuchse (1, 300) für einen Hochfrequenzgenerator (50) zum Anschluss eines elektrochirurgischen Instruments (61, 62, 63), insbesondere eines bipolaren Applikators, umfassend
- eine Koaxialbuchse (10), die ausgebildet ist, ein Koaxialsteckerelement (110) aufzunehmen, und
- eine erste Kontaktöffnung (20), die ausgebildet ist, einen Kontaktstift (210) aufzunehmen, und eine zweite Kontaktöffnung (30), die ausgebildet ist, einen Kontaktstift (210) aufzunehmen, und
- eine Positionierungsöffnung (40), die ausgebildet ist, einen Positionierungsstift aufzunehmen,
**dadurch gekennzeichnet, dass**
- die Koaxialbuchse ausgebildet ist, ein Koaxialsteckerelement (110) mit einen inneren Kontaktstift und einen äußeren Kontaktring aufzunehmen,
- die Koaxialbuchse (10), die erste Kontaktöffnung (20) und die zweite Kontaktöffnung (30) derart angeordnet sind, dass eine gerade Linie Mittelpunkte der Koaxialbuchse (10), der ersten Kontaktöffnung (20) und der zweiten Kontaktöffnung (30) verbindet,
- ein Mittelpunkt der Positionierungsöffnung (40) beabstandet zu einer geraden Linie verbindend die Mittelpunkte der Koaxialbuchse (10), der ersten Kontaktöffnung (20) und der zweiten Kontaktöffnung (30) angeordnet ist, und
- die Positionierungsöffnung (40) zwischen der ersten oder zweiten Kontaktöffnung (20, 30) und der Koaxialbuchse (10) außermittig angeordnet ist.

2. Chirurgiegeräte-Mehrfachbuchse (1, 300) gemäß Anspruch 1, **dadurch gekennzeichnet, dass** die erste und die zweite Kontaktöffnung (20, 30) benachbart zueinander angeordnet sind.

3. Chirurgiegeräte-Mehrfachbuchse (1, 300) gemäß mindestens einem der vorhergehenden Ansprüche, **dadurch gekennzeichnet, dass** ein Abstand zwischen einer Mittelachse der ersten Kontaktöffnung (20) und einer Mittelachse der zweiten Kontaktöffnung (30) um ein Vielfaches kleiner ist als
- ein Abstand zwischen der Mittelachse der ersten Kontaktöffnung (20) und einer Mittelachse der Koaxialbuchse (10), und/oder
- ein Abstand zwischen der Mittelachse der zweiten Kontaktöffnung (30) und der Mittelachse der Koaxialbuchse (10), und/oder
- ein Abstand zwischen einer Mittelachse der Positionierungsöffnung (40) und der Mittelachse der Koaxialbuchse (10),
wobei vorzugsweise der Abstand zwischen der Mittelachse der ersten Kontaktöffnung (20) und der Mittelachse der Koaxialbuchse (10) größer ist als der Abstand zwischen der Mittelachse der zweiten Kontaktöffnung (30) und der Mittelachse der Koaxialbuchse (10) und ferner vorzugsweise der Abstand zwischen der Mittelachse der zweiten Kontaktöffnung (30) und der Mittelachse der Koaxialbuchse (10) größer ist als der Abstand zwischen der Mittelachse der Positionierungsöffnung (40) und der Mittelachse der Koaxialbuchse (10).

4. Chirurgiegeräte-Mehrfachbuchse (1, 300) gemäß mindestens einem der vorhergehenden Ansprüche,
**dadurch gekennzeichnet, dass** der Abstand zwischen der Mittelachse der ersten Kontaktöffnung (20) und der Mittelachse der zweiten Kontaktöffnung (30) maximal das 5-fache eines Durchmessers der ersten und/oder zweiten Kontaktöffnung (20, 30) beträgt, und/oder
**dadurch gekennzeichnet, dass** die Mittelachse der Koaxialbuchse (10) und die Mittelachse der ersten Kontaktöffnung (20) einen Abstand von 28-29 mm, insbesondere 28,58 mm, zueinander aufweisen, und/oder
**dadurch gekennzeichnet, dass** die Mittelachse der Koaxialbuchse (10) und die Mittelachse der zweiten Kontaktöffnung (30) einen Abstand von 21-23 mm, insbesondere von 22 mm, zueinander aufweisen.

5. Chirurgiegeräte-Mehrfachbuchse (1, 300) gemäß mindestens einem der vorhergehenden Ansprüche, **dadurch gekennzeichnet, dass** die Koaxialbuchse (10) ein Anschlussende (16) aufweist, in das ein Koaxialsteckerelement (110) einführbar ist, und/oder die erste Kontaktöffnung (20) ein Anschlussende (22) aufweist, in das ein Kontaktstift (210) einführbar ist, und/oder die zweite Kontaktöffnung (30) ein Anschlussende (32) aufweist, in das ein Kontaktstift (210) einführbar ist, und/oder die Positionierungsöffnung (40) ein Anschlussende (42) aufweist, in das ein Positionierungsstift einführbar ist, wobei mindestens zwei Anschlussenden in einer gemeinsamen Ebene liegen.

6. Elektrochirurgie-Hochfrequenzgenerator (50), umfassend
- eine Chirurgiegeräte-Mehrfachbuchse (1, 300) nach mindestens einem der vorhergehenden Ansprüche 1-5.

7. Elektrochirurgiegerätestecker zum Einstecken in eine Chirurgiegeräte-Mehrfachbuchse (1, 300) nach mindestens einem der vorhergehenden Ansprüche 1-5, umfassend
- ein Koaxialsteckerelement (110) mit einem inneren Kontaktstift und einem äußeren Kontaktring, und
- mindestens einen Kontaktstift (210).

8. Elektrochirurgisches System, umfassend
- einen Hochfrequenzgenerator (50) gemäß Anspruch 6, und
- ein über die Chirurgiegeräte-Mehrfachbuchse (1, 300, 400) des Hochfrequenzgenerators angeschlossenes oder anschließbares elektrochirurgisches Instrument.

9. Kabel (65a) für ein elektrochirurgisches Instrument (64), **dadurch gekennzeichnet, dass** das Kabel mit einem Elektrochirurgiegerätestecker (70) nach Anspruch 7 ausgestattet ist.

10. Elektrochirurgisches Instrument (64), **dadurch gekennzeichnet, dass** das elektrochirurgische Instrument mit einem Kabel (65a) gemäß Anspruch 9 ausgestattet ist.

## Claims

1. A surgical device connector (1, 300) for a high-frequency generator (50) for connecting an electrosurgical instrument (61, 62, 63), in particular a bipolar applicator, comprising
- a coaxial socket (10) configured to receive a coaxial plug element (110), and
- a first contact opening (20) configured to receive a contact pin (210), and a second contact opening (30) configured to receive a contact pin (210), and
- a positioning opening (40) configured to receive a positioning pin,
**characterized in that**
- the coaxial socket (10) is configured to receive a coaxial plug element (110) having a contact pin and an outer contact ring,
- the coaxial socket (10), the first contact opening (20), and the second contact opening (30) are arranged such that a straight line connects the centers of the coaxial socket (10), the first contact opening (20), and the second contact opening (30),
- a center of the positioning opening (40) is arranged at a distance from a straight line connecting the centers of the coaxial socket (10), the first contact opening (20), and the second contact opening (30), and
- the positioning opening (40) is arranged eccentrically between the first or second contact opening (20, 30) and the coaxial socket (10).

2. A surgical device multi-port connector (1, 300) according to claim 1, **characterized in that** the first and second contact ports (20, 30) are arranged adjacent to one another.

3. A surgical device multi-port connector (1, 300) according to at least one of the preceding claims, **characterized in that** a distance between a central axis of the first contact opening (20) and a central axis of the second contact opening (30) is a multiple smaller than
- a distance between the central axis of the first contact opening (20) and a central axis of the coaxial socket (10), and/or
- a distance between the central axis of the second contact opening (30) and the central axis of the coaxial socket (10), and/or
- a distance between a central axis of the positioning opening (40) and the central axis of the coaxial socket (10),
wherein, preferably, the distance between the centerline of the first contact opening (20) and the centerline of the coaxial socket (10) is greater than the distance between the centerline of the second contact opening (30) and the centerline of the coaxial socket (10), and furthermore, preferably, the distance between the central axis of the second contact opening (30) and the central axis of the coaxial socket (10) is greater than the distance between the central axis of the positioning opening (40) and the central axis of the coaxial socket (10).

4. A multi-port surgical device connector (1, 300) according to at least one of the preceding claims,
**characterized in that** the distance between the central axis of the first contact opening (20) and the central axis of the second contact opening (30) is at most five times the diameter of the first and/or second contact opening (20, 30), and/or
**characterized in that** the central axis of the coaxial socket (10) and the central axis of the first contact opening (20) are spaced 28-29 mm, in particular 28.58 mm, apart, and/or
**characterized in that** the central axis of the coaxial socket (10) and the central axis of the second contact opening (30) are spaced 21-23 mm, in particular 22 mm, apart.

5. A multi-contact surgical device connector (1, 300) according to at least one of the preceding claims, **characterized in that** the coaxial socket (10) has a connection end (16) into which a coaxial plug element (110) can be inserted, and/or the first contact opening (20) has a connection end (22) into which a contact pin (210) can be inserted, and/or the second contact opening (30) has a connection end (32) into which a contact pin (210) can be inserted, and/or the positioning opening (40) has a connection end (42) into which a positioning pin can be inserted, wherein at least two connection ends lie in a common plane.

6. A high-frequency electrosurgical generator (50) comprising
- a surgical device multi-socket (1, 300) according to at least one of the preceding claims 1-5.

7. An electrosurgical device plug for insertion into a multiple surgical device socket (1, 300) according to at least one of the preceding claims 1-5, comprising
- a coaxial plug element (110) having an inner contact pin and an outer contact ring, and
- at least one contact pin (210).

8. An electrosurgical system comprising
- a radiofrequency generator (50) according to claim 6, and
- an electrosurgical instrument connected or connectable via the electrosurgical device multi-connector (1, 300, 400) of the high-frequency generator.

9. Cable (65a) for an electrosurgical instrument (64), **characterized in that** the cable is equipped with an electrosurgical device connector (70) according to claim 7.

10. An electrosurgical instrument (64), **characterized in that** the electrosurgical instrument is equipped with a cable (65a) according to claim 9.

## Revendications

1. Prise (1, 300) multiple d'appareils chirurgicaux d'un générateur (50) de haute fréquence pour le raccordement d'un instrument (61, 62, 63) électrochirurgical, en particulier d'un applicateur bipolaire, comprenant
- une prise (10) coaxiale, qui est constituée pour recevoir un élément (110) connecteur coaxial, et
- une première ouverture (20) de contact, qui est constituée pour recevoir une broche (210) de contact, et une deuxième ouverture (30) de contact, qui est constituée pour recevoir une broche (210) de contact, et
- une ouverture (40) de mise en position, qui est constituée pour recevoir une broche de mise en position,
**caractérisée en ce que**
- la prise coaxiale est constituée pour recevoir un élément (110) connecteur coaxial ayant une broche intérieure de contact et une bague extérieure de contact,
- la prise (10) coaxiale, la première ouverture (20) de contact et la deuxième ouverture (30) de contact sont disposées, de manière à ce qu'une ligne droite passe par les points milieux de la prise (10) coaxiale, de la première ouverture (20) de contact et de la deuxième ouverture (30) de contact,
- un point milieu de l'ouverture (40) de mise en position est disposé à distance d'une ligne droite passant par les points milieux de la prise (10) de contact, de la première ouverture (20) de contact et de la deuxième ouverture (30) de contact, et
- l'ouverture (40) de mise en position est disposée hors du milieu entre la première ou la deuxième ouverture (20, 30) de contact et la prise (10) coaxiale.

2. Prise (1, 300) multiple d'appareils chirurgicaux suivant la revendication 1, **caractérisée en ce que** la première et la deuxième ouvertures (20, 30) de contact sont disposées en étant voisines l'une de l'autre.

3. Prise (1, 300) multiple d'appareils chirurgicaux suivant au moins l'une des revendications précédentes, **caractérisée en ce qu'**une distance entre un axe médian de la première ouverture (20) de contact et un axe médian de la deuxième ouverture (30) de contact est plus petite d'un multiple qu'
- une distance entre l'axe médian de la première ouverture (20) de contact et un axe médian de la prise (10) coaxiale, et/ou
- une distance entre l'axe médian de la deuxième ouverture (30) de contact et l'axe médian de la prise (10) coaxiale, et/ou
- une distance entre un axe médian de l'ouverture (40) de mise en position et l'axe médian de la prise (10) coaxiale,
dans laquelle, de préférence, la distance entre l'axe médian de la première ouverture (20) de contact et l'axe médian de la prise (10) coaxiale est plus grande que la distance entre l'axe médian de la deuxième ouverture (30) de contact et de l'axe médian de la prise (10) coaxiale et de préférence, en outre, la distance entre l'axe médian de la deuxième ouverture (30) de contact et l'axe médian de la prise (10) coaxiale est plus grande que la distance entre l'axe médian de l'ouverture (40) de mise en position et l'axe médian de la prise (10) coaxiale.

4. Prise (1, 300) multiple d'appareils chirurgicaux suivant au moins l'une des revendications précédentes, **caractérisée en ce que** la distance entre l'axe médian de la première ouverture (20) de contact et l'axe médian de la deuxième ouverture (30) de contact représente au maximum 5 fois un diamètre de la première et/ou de la deuxième ouverture (20, 30) de contact, et /ou **caractérisée en ce que** l'axe médian de la prise (10) coaxiale et l'axe médian de la première ouverture (20) de contact ont l'un par rapport à l'autre une distance de 28-29 mm, en particulier de 28,58 mm, et/ou **caractérisée en ce que** l'axe médian de la prise (10) coaxiale et l'axe médian de la deuxième ouverture (30) de contact ont l'un par rapport à l'autre une distance de 21-23 mm, en particulier de 22 mm.

5. Prise (1, 300) multiple d'appareils chirurgicaux suivant au moins l'une des revendications précédentes, **caractérisée en ce que** la prise (10) coaxiale a une extrémité (16) de connexion, dans laquelle un élément (110) connecteur coaxial peut être introduit et/ou la première ouverture (20) de contact a une extrémité (22) de connexion, dans laquelle une broche (210) de contact peut être introduite et/ou la deuxième ouverture (30) de contact a une extrémité (32) de connexion, dans laquelle une broche (210) de contact peut être introduite et/ou l'ouverture (40) de mise en position a une extrémité (42) de connexion, dans laquelle une broche de mise en position peut être introduite, dans laquelle au moins deux extrémités de connexion sont dans un plan commun.

6. Générateur (50) de haute fréquence d'électrochirurgie, comprenant
- une prise (1, 300) multiple d'appareils chirurgicaux suivant au moins l'une des revendications 1 à 5 précédentes.

7. Connecteur d'appareils chirurgicaux à enficher dans une prise (1, 300) multiple d'appareils chirurgicaux suivant au moins l'une des revendications 1 à 5 précédentes, comprenant
- un élément (110) connecteur coaxial ayant une broche intérieure de contact et une bague extérieure de contact, et
- au moins une broche (210) de contact.

8. Système électrochirurgical, comprenant
- un générateur (50) de haute fréquence suivant la revendication 6, et
- un instrument électrochirurgical raccordé par la prise (1, 300, 400) multiple d'appareils chirurgicaux du générateur haute fréquence ou pouvant l'être.

9. Câble (65a) d'un instrument (64) électrochirurgical, **caractérisé en ce que** le câble est équipé d'un connecteur (70) d'appareils chirurgicaux suivant la revendication 7.

10. Instrument (64) électrochirurgical, **caractérisé en ce que** l'instrument électrochirurgical est équipé d'un câble (65a) suivant la revendication 9.
